# EUROPEAN PATENT APPLICATION

(11) **EP 2 532 373 A1**
(43) Date of publication of application: **12.12.2012**
(21) Application number: 11739750.5
(22) Date of filing: 01.02.2011
(51) Int. Cl.: A61L 31/00

(54) **BIOCOMPATIBLE DEVICE**

(30) Priority: 03.02.2010 JP 2010022565
(71) Applicant: National Institute for Materials Science, Tsukuba-shi, Ibaraki 305-0047 (JP); The University of Tokyo, Bunkyo-Ku Tokyo 113-8654 (JP)
(72) Inventor: TAGUCHI Tetsushi, Tsukuba-shi Ibaraki 305-0047 (JP); KATADA Yasuyuki, Tsukuba-shi Ibaraki 305-0047 (JP); NAGAI Ryozo, Tokyo 113-8654 (JP); MANABE Ichiro, Tokyo 113-8654 (JP); FUJIO Katsuhito, Tokyo 113-8654 (JP)
(74) Representative: Calamita, Roberto
(86) International application number: PCT/JP2011/052058
(87) International publication number: WO 2011/096402

(57) **Abstract**

Disclosed is a biocompatible device surface-coated on the base material thereof with a biocompatible polymer layer having antithrombogenicity and endothelialization activity, and embedded in or attached to a living body for use. The polymer layer comprises a polymer matrix formed by the crosslinking of a cell-adhesive peptide-containing polymer.

## Description

### Technical Field

The present invention relates to a biocompatible device surface-coated on the base material thereof with a biocompatible polymer layer having antithrombogenicity and endothelialization activity, and used by being embedded in a living body or in contact with the blood.

### Background Art

Stents, a type of medical apparatus used in contact with the blood, are used for the treatment of cardiovascular diseases such as ischemic heart disease and dissecting aortic aneurysm. A stent placement in coronary artery represents a major treatment of ischemic heart disease. However, 20 to 40% of cases involves restenosis, a re-narrowing of the once dilated vascular lumen. The need for another revascularization procedure thus represents the biggest problem of stent placement. As a countermeasure against the drawback associated with restenosis, drug-eluting stents that provide the sustained-release of a drug from the coronary artery stent surface have been developed, and used in the clinic. For sustained drug release, the existing drug-eluting stents use a synthetic polymer matrix, polylactic acid or polyglycolic acid that yields an acid upon decomposition, polycaprolactone, and copolymers of these. Because of this, the blood vessel wall undergoes a persistent wound healing, and endothelialization by the vascular endothelial cells does not take place in the stent lumen, resulting in a stent thrombosis (see Patent Documents 1 and 2). Another drawback is the need to take an anti-platelet drug--a substance with a very high incidence of side effects-for 6 months to 1 year, and aspirin for life, in order to prevent thrombosis.

As described above, there is a limit to the surface modification of a medical apparatus used in contact with the blood for extended time periods, and there have been attempts to utilize the antithrombogenicity of vascular endothelial cells. This is based on the realization that blood clots develop in portions of the blood vessels in the body where the vascular endothelial cells have detached, but not in portions where vascular endothelium is present.

Broadly, two techniques are available for the preparation of a medical apparatus that has a vascular endothelial cell layer. In one technique, transplantation is performed after forming an inner coating by inoculating vascular endothelial cells in advance on the inner surface of a medical apparatus. A drawback of this technique, however, is that it requires procedures such as collecting and culturing cells, and cannot be used in emergency situations, aside from that the cell collection places a heavy burden on patients. In the other technique, a substance that promotes adhesion or proliferation of vascular endothelial cells soon after the transplantation is immobilized on a surface of the medical apparatus used in contact with the blood. Examples of the proteins considered in this technique include extracellular matrix proteins such as adhesive peptides, collagens, and fibronectins, and growth factors that promote proliferation of vascular endothelial cells. Patent Documents 3, 4, 5, 6, 7, and 8 disclose techniques for promoting adhesion or proliferation of vascular endothelial cells. In one technique, peptide sequences associated with cell adhesion, or growth factors are immobilized on a polymer base material by covalent bonding through introduction of carboxyl groups by the graft polymerization of acrylic acid or the like. In another technique, a composite material is used that is prepared by using a porous polymer with extracellular matrix proteins or growth factors.

While the techniques are found to be effective in terms of the surface adhesion and proliferation of vascular endothelial cells, there is a drawback that in the materials obtained as above the effect needed to suppress the blood clotting that occurs immediately after the medical apparatus is embedded is not considered. Accordingly, there is a demand for the development of a biocompatible device coated with a polymer layer that exhibits antithrombogenicity immediately after the transplantation, and that has endothelialization activity after the transplantation.

### Citation List

### Patent Document

Patent Document 1: JP-A-8-33718
Patent Document 2: JP-A-9-56807
Patent Document 3: JP-A-10-137334
Patent Document 4: JP-A-5-76588
Patent Document 5: JP-T-11-504548 (the term "JP-T" as used herein means a published Japanese translation of a PCT patent application)
Patent Document 6: JP-T-2001-502187
Patent Document 7: JP-T-2005-503240
Patent Document 8: JP-A-2006-68401

### Summary of Invention

### Problems that the Invention is to Solve

It is an object of the present invention to provide a biocompatible device surface-coated with a biocompatible polymer layer having antithrombogenicity and endothelialization activity, and a medical apparatus using the biocompatible device, among others.

### Means for Solving the Problems

Invention 1 is a biocompatible device surface-coated on the base material thereof with a biocompatible polymer layer having antithrombogenicity and endothelialization activity, and embedded in or attached to a living body for use, wherein the polymer layer comprises a polymer matrix formed by the crosslinking of a cell-adhesive peptide-containing polymer.

Invention 2 is a biocompatible device according to invention 1, wherein the polymer matrix is formed by the crosslinking of the polymer via a citric acid derivative with active ester groups.

Invention 3 is a biocompatible device according to invention 2, wherein the citric acid derivative with active ester groups is trisuccinimidyl citrate or trisulfosuccinimidyl citrate.

Invention 4 is a biocompatible device according to any one of inventions 1 to 3, wherein the cell-adhesive peptide-containing polymer is one or a combination of two or more selected from gelatin, alkali-treated gelatin, acid-treated gelatin, collagen, atelocollagen, alkali-treated collagen, fibrinogen, keratin, fibroin, laminin, fibronectin, vitronectin, and a derivative thereof.

Invention 5 is a biocompatible device according to any one of inventions 1 to 4, wherein the cell-adhesive peptide represents one of or a combination of two or more of the peptide sequences selected from arginine-glycine-aspartic acid (RGD), tyrosine-isoleucine-glycine-serine-arginine (YIGSR), and isoleucine-lycine-valine-alanine-valine (IKVAV).

Invention 6 is a biocompatible device according to any one of inventions 1 to 5, wherein the base material is one or a composite material of two or more selected from a polymer material, a metallic material, a ceramic material, a nonwoven fabric, and a biological tissue.

Invention 7 is a biocompatible device according to invention 6, wherein the base material is a polymer material, and wherein the polymer material is one or a combination of two or more selected from polyethylene, polypropylene, polytetrafluoroethylene, polystyrene, polyurethane, silicone, polylactic acid, polyglycolic acid, poly ε-caprolactone, a polylactic acid-glycolic acid copolymer, a poly ε-caprolactone-glycolic acid copolymer, and a polylactic acid-poly ε-caprolactone copolymer.

Invention 8 is a biocompatible device according to invention 6, wherein the base material is a metallic material, and wherein the metallic material is one or a combination of two or more selected from SUS316L stainless steel, a cobalt-chromium alloy, nickel-free high-nitrogen stainless steel, a magnesium alloy, and a shape-memory alloy.

Invention 9 is a biocompatible device according to invention 6, wherein the base material is a ceramic material, and wherein the ceramic material is one or a combination of two or more selected from a hydroxyapatite sintered body, low crystalline hydroxyapatite, β-tricalcium phosphate, and α-tricalcium phosphate.

Invention 10 is a biocompatible device according to any one of inventions 1 to 9, wherein the polymer layer is formed on a base material surface surface-treated with an acid, an alkali, or an organic solvent.

Invention 11 is a biocompatible device according to any one of inventions 1 to 10, wherein a drug is impregnated in the polymer matrix.

Invention 12 is a biocompatible device according to invention 11, wherein the drug is one or a combination of two or more selected from a cellular differentiation inducer, an anticancer agent, an immunosuppresant, a cell growth factor, a cytokine, a thrombin inhibitor, an antithrombogenic drug, a thrombolytic agent, a fibrinolytic drug, a vasospasm inhibitor, a calcium channel blocker, a vasodilating drug, a high blood pressure drug, an antimicrobial drug, an antibiotic, a surface glycoprotein receptor inhibitor, an anti-platelet drug, an antimitotic drug, a microtubule inhibitor, an antisecretory drug, an actin inhibitor, a remodeling inhibitor, an antisense·nudeotide, an antimetabolite, an antiproliferative substance, an anti-cancer chemotherapy drug, an anti-inflammatory steroid or a nonsteroidal anti-inflammatory drug, an immunosuppresant, a growth hormone antagonist, a growth factor, a dopamine·agonist, a radiotherapeutic agent, a peptide, a protein, an enzyme, an extracellular matrix component, an inhibitor, a free radical·scavenger, a chelating agent, an antioxidizing agent, an anti-polymerase, an anti-viral drug, a photodynamic therapeutic drug, and a gene therapy drug.

Invention 13 is a biocompatible device according to invention 12, wherein the cellular differentiation inducer is tamibarotene.

Invention 14 is a medical apparatus embedded in or attached to a living body for use in a medical procedure, the medical apparatus comprising the biocompatible device of any one of inventions 1 to 13 configured to have a structure suited for the medical procedure.

Invention 15 is a stent inserted into a blood vessel of a living body to dilate the blood vessel from inside, the stent comprising the biocompatible device of any one of inventions 1 to 14 configured to have the structure of the stent.

Invention 16 is a biocompatible device according to invention 10, wherein the acid is aqua regia.

Invention 17 is a biocompatible device according to invention 4, wherein the cell-adhesive peptide-containing polymer is hydrophobically modified.

Invention 18 is a biocompatible device producing method that comprises coating a surface of a base material with a coating solution that contains a cell-adhesive peptide-containing polymer and a crosslinker, so as to form a polymer layer having antithrombogenicity and endothelialization activity.

Invention 19 is a biocompatible device producing method according to invention 18, wherein the coating is performed multiple times.

Invention 20 is a biocompatible device producing method according to invention 18, wherein the coating solution contains a drug.

Invention 21 is a biocompatible device producing method according to invention 18, further comprising hydrophobically-modified cell-adhesive peptide-containing polymer.

Invention 22 is a biocompatible device producing method according to invention 18, wherein the coating solution contains a drug, and wherein the method further comprises adjusting the concentration of the crosslinker in the coating solution in a range of from 5 mM to 200 mM according to the desired drug sustained-release from the biocompatible device.

### Advantage of the Invention

In the present invention, the biocompatible polymer layer is configured from the polymer matrix obtained by polymer crosslinking. The base material can thus be coated with the polymer layer by covalent bonding, intermolecular interaction, or mechanical anchoring effect.

The polymer layer thus allows for the use of the cell-adhesive peptide-containing polymer, and enables the surface properties of the biocompatible device to be converted to properties that include both antithrombogenicity and endothelialization activity. The polymer layer also provides a restenosis suppressing effect, whereby abnormal proliferation of the vascular smooth muscle cells can be suppressed.

Specifically, as recited in inventions 2 and 3, the citric acid derivative with active ester groups used as the crosslinker of the cell-adhesive peptide-containing polymer makes it possible to provide a biocompatible polymer layer coating having antithrombogenicity and endothelialization activity.

Further, because the polymer layer configured as above can include a low-molecular compound such as a drug in the polymer matrix for sustained-release, materials useful for further enhancing the foregoing effects but unsuited to provide a polymer matrix structure can also be used to improve the function of a biocompatible device.

Further, the foregoing functions of the present invention are applicable to a stent, and the invention can thus provide a stent having all of antithrombogenicity, endothelialization, and a restenosis suppressing effect, considered not possible in the past.

### Brief Description of Drawings

Fig. 1 is a photograph representing the external appearance of SNo.1-01 of Table 1.
Fig. 2 is a photograph representing the external appearance of SNo.1-04 of Table 1.
Fig. 3 is a photograph representing the external appearance of SNo.1-05 of Table 1.
Fig. 4 is a photograph representing the external appearance of SNo.1-06 of Table 1.
Fig. 5 is a photograph representing the external appearance of SNo.1-07 of Table 1.
Fig. 6 is an electron micrograph of SNo.1-07 of Table 1.
Fig. 7 is a photograph representing the external appearance of SNo.1-08 of Table 1.
Fig. 8 is an electron micrograph of SNo.1-08 of Table 1.
Fig. 9 is a photograph representing the external appearance of SNo.1-09 of Table 1.
Fig. 10 is an electron micrograph of SNo.1-09 of Table 1.
Fig. 11 is a photograph representing the external appearance of SNo.1-10 of Table 1.
Fig. 12 is a photograph representing the external appearance of SNo.1-13 of Table 1.
Fig. 13 is a photograph representing the external appearance of SNo.1-14 of Table 1.
Fig. 14 is a photograph representing the external appearance of SNo.1-15 of Table 1.
Fig. 15 is a photograph representing the external appearance of SNo.1-16 of Table 1.
Fig. 16 is an electron micrograph of SNo.1-16 of Table 1.
Fig. 17 is a photograph representing the external appearance of SNo.1-17 of Table 1.
Fig. 18 is an electron micrograph of SNo.1-17 of Table 1.
Fig. 19 is a photograph representing the external appearance of SNo.1-18 of Table 1.
Fig. 20 is an electron micrograph of SNo.1-18 of Table 1.
Fig. 21 is a photograph representing the external appearance of SNo.1-19 of Table 1.
Fig. 22 is an electron micrograph of SNo.1-19 of Table 1.
Fig. 23 is a photograph representing the external appearance of SNo.1-20 of Table 1.
Fig. 24 is an electron micrograph of SNo.1-20 of Table 1.
Fig. 25 is a photograph showing the inner surface of the stent SNo.1-20 of Table 1 after the placement in a pig coronary artery.
Fig. 26 is an electron micrograph showing the inner surface of the stent SNo.1-20 of Table 1 after the placement in a pig coronary artery.
Fig. 27 is a photograph showing the external appearance of the stent SNo.1-23 of Table 1 after the evaluation in an antithrombogenicity test.
Fig. 28 is a sample electron micrograph of the stent SNo.1-23 of Table 1 after the evaluation in an antithrombogenicity test.

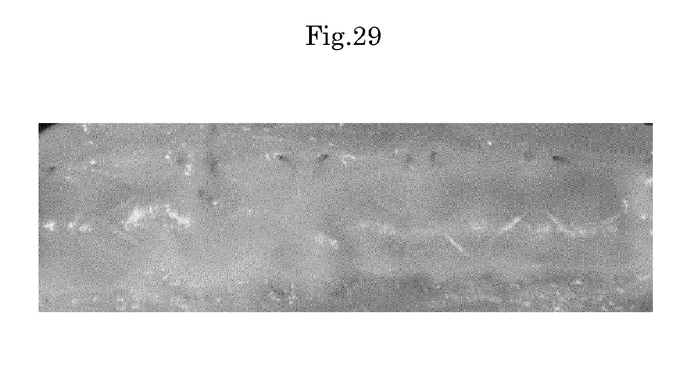

Fig. 29 is a photograph showing the inner surface of the stent SNo.1-26 of Table 1 after the placement in a pig coronary artery.

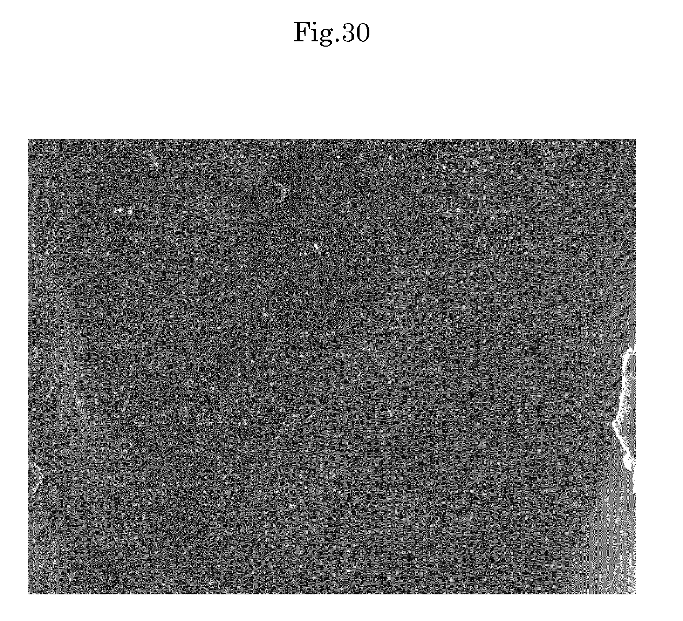

Fig. 30 is an electron micrograph showing the inner surface of the stent SNo.1-26 of Table 1 after the placement in a pig coronary artery.
Fig. 31 is a photograph showing the external appearance of the stent SNo.1-27 of Table 1 after the evaluation in an antithrombogenicity test.
Fig. 32 is an electron micrograph of the stent SNo.1-27 of Table 1 after the evaluation in an antithrombogenicity test.
Fig. 33 is a photograph showing the external appearance of the stent SNo.1-28 of Table 1 after the evaluation in an antithrombogenicity test.
Fig. 34 is an electron micrograph of the stent SNo.1-28 of Table 1 after the evaluation in an antithrombogenicity test.
Fig. 35 is a photograph showing the external appearance of the stent SNo.1-29 of Table 1 after the evaluation in an antithrombogenicity test.
Fig. 36 is an electron micrograph of the stent SNo.1-29 of Table 1 after the evaluation in an antithrombogenicity test.
Fig. 37 is a photograph showing the inner surface of the stent SNo.1-30 of Table 1 after the placement in a pig coronary artery (Comparative Example).
Fig. 38 is an electron micrograph showing the inner surface of the stent SNo.1-30 of Table 1 after the placement in a pig coronary artery (Comparative Example).
Fig. 39 is a photograph showing the external appearance of the disc SNo.1-04 of Table 1 after a vascular endothelial cell adhesion test.
Fig. 40 is a photograph showing the external appearance of the disc SNo.1-06 of Table 1 after a vascular endothelial cell adhesion test.
Fig. 41 is a photograph showing the external appearance of the stent SNo.1-13 of Table 1 after a vascular endothelial cell adhesion test.
Fig. 42 is a photograph showing the external appearance of the stent SNo.1-15 of Table 1 after a vascular endothelial cell adhesion test.
Fig. 43 represents ATR-IR spectra of SUS316L stainless steel surfaces treated with the acetone (SNo.5-01), NaOH (SNo.5-02), and diluted aqua regia (SNo.5-03) of Table 5.
Fig. 44 is a diagram representing the result of forming a coating on a stent surface multiple times.
Fig. 45 represents SEM images of stent surfaces and stent cross sections after form ing a coating.
Fig. 46 is a diagram representing an example of a retinoylated gelatin synthesis scheme.

### Mode for Carrying Out the Invention

The present invention is described in detail below.

The base material used in the present invention may be, for example, one or a composite material of two or more selected from a polymer material, a metallic material, a ceramic material, a nonwoven fabric, and a biological tissue. Specifically, any material may be used, as long as a polymer layer can be immobilized on the base material surface by covalent bonding, intermolecular interaction, or mechanical anchoring effect. In a biological tissue, biopolymers with an amino group, such as collagen and laminin, found as extracellular matrix components can undergo reaction with citric acid derivative with active ester groups. Immobilization is thus possible by the reaction of the amino group with the active ester.

Examples of the polymer material usable for the base material include polyethylene, polypropylene, polytetrafluoroethylene, polystyrene, polyurethane, silicone, polylactic acid, polyglycolic acid, poly ε-caprolactone, a polylactic acid-glycolic acid copolymer, a poly ε-caprolactone-glycolic acid copolymer, and a polylactic acid-poly ε-caprolactone copolymer. These materials allow for introduction of an amino group to the surface, or introduction of irregularities with the use of a file or the like, and thus enable a polymer layer to be immobilized on the base material surface by covalent bonding, intermolecular interaction, or mechanical anchoring effect.

Examples of the metallic material include SUS316L stainless steel, a cobalt-chromium alloy, nickel-free high-nitrogen stainless steel, a magnesium alloy, and a shape-memory alloy. These metallic materials have a surface hydroxyl group that can react with a citric acid derivative with active ester groups, and thus enable a polymer layer to be immobilized on the base metal surface by covalent bonding or intermolecular interaction.

The ceramic material may be, for example, one or a combination of two or more selected from a hydroxyapatite sintered body, low crystalline hydroxyapatite, β-tricalcium phosphate, and α-tricalcium phosphate. These ceramic materials have a surface hydroxyl group that can react with a citric acid derivative with active ester groups, and thus enable a polymer layer to be immobilized on the base metal surface by covalent bonding or intermolecular interaction.

The base material surface may be surface treated with an acid, an alkali, or an organic solvent before being coated. In this way, detachment strength can be improved, as will be described in detail in Example 3 below.

Any polymer matrix can be used in the present invention, as long as it contains cell-adhesive peptides. In addition to the materials used in Examples, the following materials also may be used.

Preferably, the polymer layer is a material which is obtained by mixing the main polymer with other polymer materials or with low-molecular organic compounds such as drugs and subjecting the main polymer to the crosslinking reaction with a citric acid derivative with active ester groups, and in which the polymer matrix is combined with the drug at the molecular level.

Using the cell-adhesive peptide-containing polymer as the main material of the polymer matrix enables the cell-adhesive peptides to be concentrated by the crosslinking with the citric acid derivative with active ester groups, and allows for introduction of the citric acid-derived carboxyl group. A polymer layer coating can thus be formed that has endothelialization activity and antithrombogenicity.

Preferably, the cell-adhesive peptide-containing polymer in the coating is collagen, atelocollagen, alkali-treated collagen, gelatin, acid-treated gelatin, alkali-treated gelatin, keratin, serum albumin, egg white albumin, genetically recombined albumin, hemoglobin, casein, globulin, fibrinogen, or a derivative of these.

Of these, for example, alkali-treated collagen, alkali-treated gelatin, and derivatives of these are more preferred. Desirably, these materials contain an amino group within the molecule, and are suited for the crosslinking reaction with a citric acid derivative with active ester groups.

The cell-adhesive peptide-containing polymer is superior to polymers that do not contain cell-adhesive peptides, as clearly demonstrated by the cell-adhesive peptide-containing collagens and gelatins (Table 1 in Example 1) that, with the cell-adhesive peptides, provide endothelialization activity for the coated base material.

Superiority over the polymers containing no cell-adhesive peptide is also demonstrated when the cell-adhesive peptides represent one of or a combination of two or more of the peptide sequences selected from arginine-glycine-aspartic acid (RGD), tyrosine-isoleucine-glycine-serine-arginine (YIGSR), and isoleucine-lycine-valine-alanine-valine (IKVAV), as can be clearly seen from Table 1 of Example 1 in which the collagens and gelatins, with the molecular sequences such as RGD, provide endothelialization activity for the coated base material.

The concentration of the polymer used for the preparation of the polymer matrix is not particularly limited, and is preferably 7.5 to 30 mass%, more preferably 15 mass% ± 6 mass%, and further preferably 15 mass% ± 3 mass%. An excessively low polymer concentration makes it difficult to maintain a crosslinked structure of high crosslinking density, and it becomes difficult to obtain the polymer matrix. The concentration ratio of the polymer for preparing the polymer matrix to the citric acid derivative with active ester groups in a mixed solution thereof should be 3 (mass%) to 4 (mM). For example, the concentration of the citric acid derivative with active ester groups is preferably 20 mM for a 15 mass% polymer concentration. A citric acid derivative with active ester groups concentration below 20 mM for a 15 mass% polymer concentration makes it difficult to obtain the antithrombogenicity effect. Further, under the same polymer concentration condition, a citric acid derivative with active ester groups concentration at or above 20 mM makes it difficult to maintain a crosslinked structure of high crosslinking density as the citric acid derivative with active ester groups concentration increases from 20 mM, and it becomes difficult to obtain the polymer matrix.

Citric acid derivatives with active ester groups are desirable as the crosslinker for the coating material polymer matrix. Using citric acid derivatives with active ester groups produced more desirable results than when citric acid derivatives with active ester groups were not used, as demonstrated in SNo.1-17 and 1-27 of Examples, in which the use of the citric acid derivative with active ester groups provided antithrombogenicity and endothelialization activity.

The citric acid derivative with active ester groups may be trisuccinimidyl citrate or tri(sulfosuccinimidyl)citrate, or a combination of these. These materials also can be used as they react with an amino group to produce the polymer layer.

Further, crosslinking the cell-adhesive peptide-containing polymers with the citric acid derivative with active ester groups produces more desirable results in terms of antithrombogenicity and endothelialization activity, as is clear from Table 1 of Example 1.

The citric acid derivative with active ester groups concentration suited for obtaining the polymer matrix is preferably 5 to 200 mM, more preferably 5 to 100 mM, further preferably 5 to 40 mM, though it depends on the concentration of the polymer used for the preparation of the polymer matrix.

A deficiency of the citric acid derivative with active ester groups results in a fewer crosslinking points in the polymer forking the polymer matrix, and the polymer matrix structure cannot be maintained. The excess citric acid derivative with active ester groups causes the binding of the individual polymers to one or two of the carboxyl groups of the citric acid, and disables crosslinking. The result is the reduced number of crosslinking points, and the failure to maintain the structure.

The crosslinking reaction temperature is preferably from 15°C to 37°C, more preferably 15°C to 30°C, further preferably ordinary temperature. Reaction at the excessively high temperatures increases the reaction rate, and formation of a uniform coating becomes difficult. On the other hand, reaction at the excessively low temperatures freezes the solvent, and the reaction does not easily proceed.

The reaction time is preferably within 24 hours at room temperature (25°C). For example, when the citric acid derivative with active ester groups is added in 20 mM with respect to the total reaction solution, the polymer matrix is formed in 10 to 20 minutes at room temperature.

The drugs are preferably low-molecular compounds poorly soluble in water. Further, the drugs may be those that can be incorporated in the polymer layer by using the same practice. Examples include cellular differentiation inducers, anticancer agents, immunosuppresants, cell growth factors, cytokines, thrombin inhibitors, antithrombogenic drugs, thrombolytic agents, fibrinolytic drugs, vasospasm inhibitors, calcium channel blockers, vasodilating drugs, high blood pressure drugs, antimicrobial drugs, antibiotics, surface glycoprotein receptor inhibitors, anti-platelet drugs, antimitotic drugs, microtubule inhibitors, antisecretory drugs, actin inhibitors, remodeling inhibitors, antisense·nucleotides, antimetabolites, antiproliferative substances, anti-cancer chemotherapy drugs, anti-inflammatory steroids or nonsteroidal anti-inflammatory drugs, immunosuppresants, growth hormone·antagonists, growth factors, dopamine·agonists, radiotherapeutic agents, peptides, proteins, enzymes, extracellular matrix components, inhibitors, free radical·scavengers, chelating agents, antioxidizing agents, anti-polymerases, anti-viral drugs, photodynamic therapeutic drugs, and gene therapy drugs. These may be used either alone or in a combination of two or more. The cellular differentiation inducers are preferably poorly water-soluble tamibarotene. The anticancer agents are preferably poorly water-soluble paclitaxel and derivatives thereof. The immunosuppresants are preferably poorly water-soluble sirolimus and derivatives thereof.

The solvent that can be used for the preparation of the coating material is one that can dissolve the polymer used for preparing the polymer matrix, and the citric acid derivative with active ester groups, and that does not cause chemical decomposition of the added materials. Non-protonic polar solvents are preferred. Examples include dimethylsulfoxide (DMSO), N,N-dimethylformamide, N-methylpyrrolidone, and 1,1,1,3,3,3-hexafluoroisopropanol.

The reaction products (N-hydroxysuccinimide, N-hydroxysulfosuccinimide) and the unreactants that generate during the formation of the polymer matrix of the polymer matrix-forming polymer and the citric acid derivative with active ester groups can be removed by dipping the resulting polymer matrix in deionized water.

The displacement of the organic solvent contained in the polymer matrix with deionized water requires suppressing the dissolving of the included added materials (such as low-molecular organic compounds), and the hydrolysis of the polymer. From this standpoint, the displacement of the solvent with deionized water should be performed at preferably 0 to 20°C, more preferably 0 to 10°C, further preferably 0 to 5°C.

A specific means to prepare the mixed solution for producing the coating material is not particularly limited. For example, a stirring device such as a small mixer is preferably used to prepare a thorough uniform mixture.

The present invention is described in more detail below using Examples. It should be noted that the present invention is in no way limited by the following Examples.

### Example 1

In Example 1, the coating material is specifically described.

### [Coating of Base Material with Polymer Layer]

Tamibarotene (Am80) used as a drug was mixed with a 7.5, 15, 30% alkali-treated gelatin (pig skin-derived) or alkali-treated collagen (pig skin-derived)/10% lactic acid-DMSO solution (1 ml) to make the final drug concentration 0,35, 245, or 700 mM. Then, a 10% lactic acid-DMSO solution (250 µl) of trisuccinimidyl citrate (TSC) was added in the final concentration of 10, 20, or 40 mM to the mixture in a 5-ml tube. The resulting mixture was stirred for 30 seconds, and degassed by centrifugation for 30 seconds to prepare a polymer coating solution. A base material was then dipped in the polymer coating solution for 10 seconds.

Discs and stents were used as the base materials.

The disc-shaped base materials were 1 mm in thickness and 10 to 12 mm in diameter, and used the following materials, as presented in Table 1.
SUS* (SUS316L (C: 0.03% or less, Si: 1% or less, Mn: 2% or less, P: 0.045% or less, S: 0.03% or less, Ni: 12 to 15%, Mo: 2 to 3%)
HNS* (high-nitrogen stainless steel (23Cr-1 Mo-1 N))
CoCr* (CoCr alloy (65Co-29Cr-6Mo)
HAP* (sintered hydroxyapatite)
Acryl* (acryl resin (Sunday Sheet; Acrylsunday)
These were used after roughening the surface with a file.
PTEE* ((polyethylene terephthalate (PTFE) nonwoven fabric, Yamakatsu Labo Co., Ltd.) was also used as the material of the disc-shaped base material.

The stents had a length of 10 mm and a diameter of 1.4 mm, and were made of SUS*, CoCr*, and HNS*.

Each disc-shaped base material sample with a 10-mm diameter was placed in a 25-ml conical tube, and centrifuged (3,500 rpm, 1 min) to form a uniform coating layer. Coatings were also formed for other disc-shaped samples by using the same method. The samples were air-dried overnight at room temperature, dipped in 4°C ultrapure water for 8 hours, and dried overnight in a desiccator at room temperature to obtain disc-shaped samples having coating layers.

As for the stents, each sample was dipped in a polymer coating solution for 10 sec, and inserted into a cut injection needle. The excess reaction solution at the edge portions of the stent was then removed by centrifugation (6,000 rpm, 10 sec). Then, the stent was dipped in 4°C ultrapure water in a 15-mL centrifuge tube, and lactic acid, DMSO, and the by-product N-hydroxysuccinimide were removed. The ultrapure water was exchanged every two hours, five times each day. After exchanging the water for 3 days, the sample was air-dried for a whole day, and dried in a desiccator for a day to obtain a stent with a polymer layer coating.

A CoCr stent sample coated with atelocollagen (C*) dissolved in 0.01 N-HCl was also prepared as a control using the same procedure (1-27 in Table 1).

### [Antithrombogenicity Evaluation of Polymer Layer Coated Base Materials]

Each of the samples obtained as above was dipped in the fresh blood (about 1 mL) collected from a rat, and incubated for 15 to 30 min (37°C). After being washed with 0.1 M phosphate buffer (PBS) three times, the sample was observed for formation of a blood clot using a stereom icroscope and an electron microscope.

The effects of the polymer layer coating of the present invention on the base material surfaces were evaluated for SNo.1-01, 1-04 to 1-10, 1-13 to 1-20, 1-23, and 1-27 to 1-29.

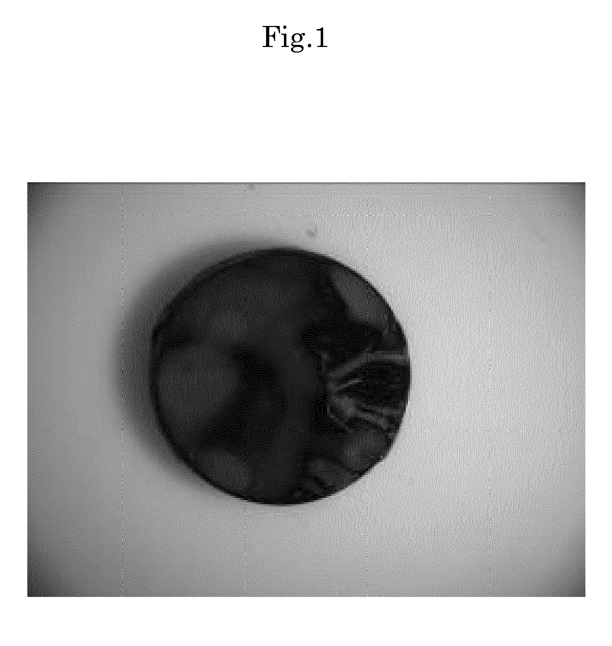

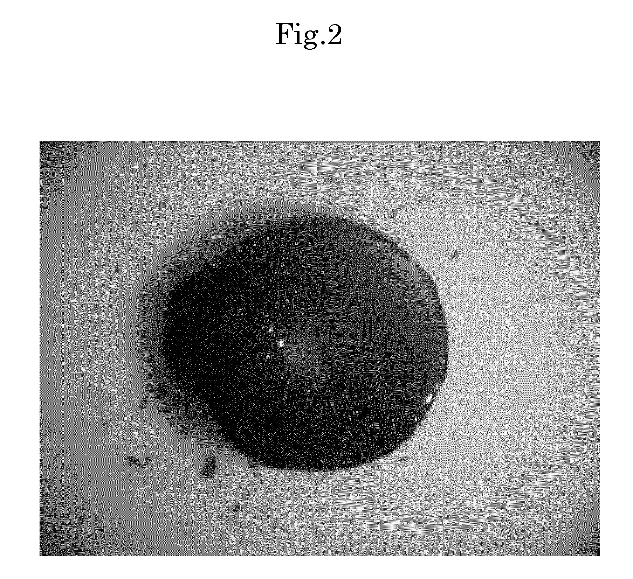

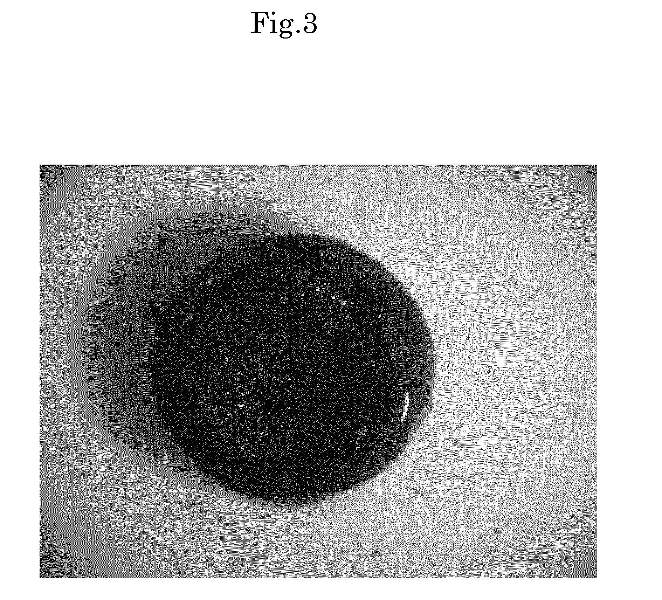

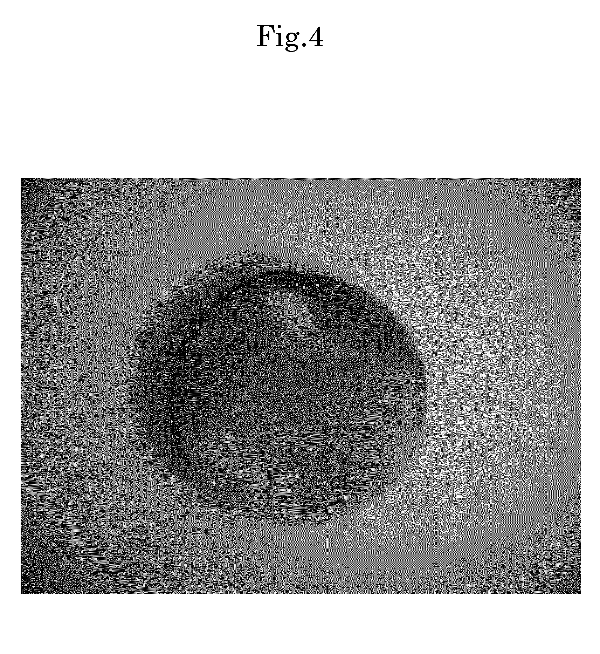

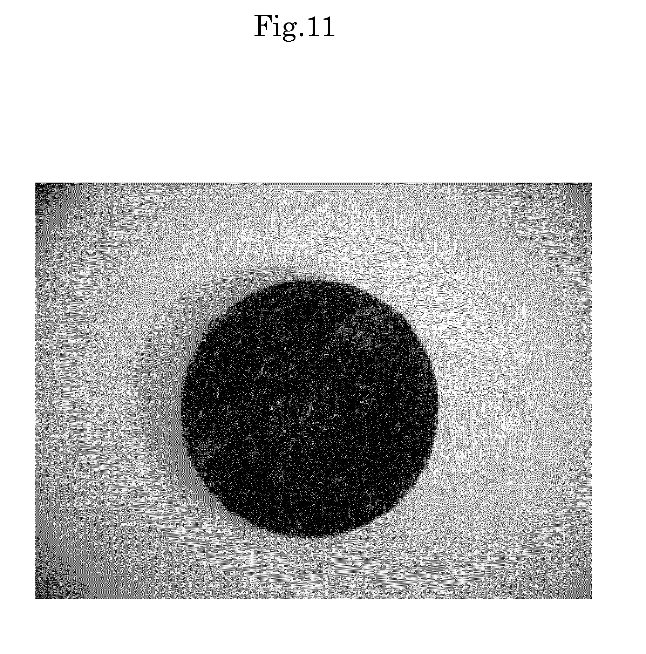

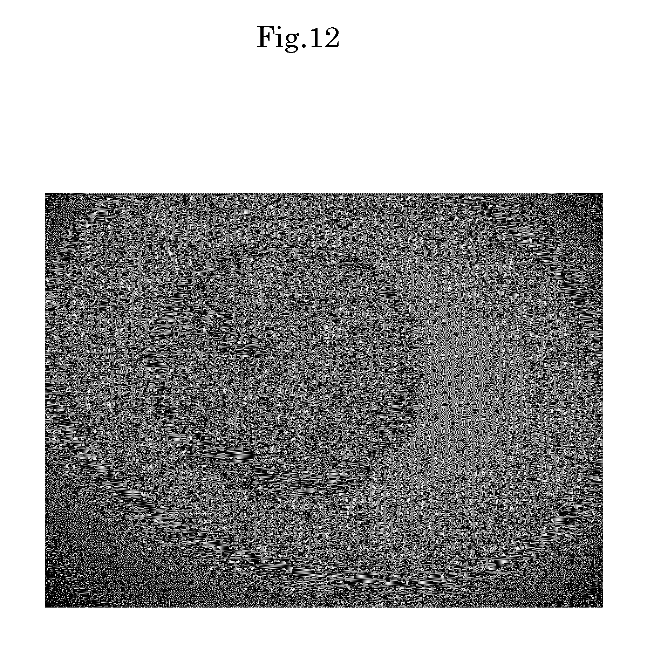

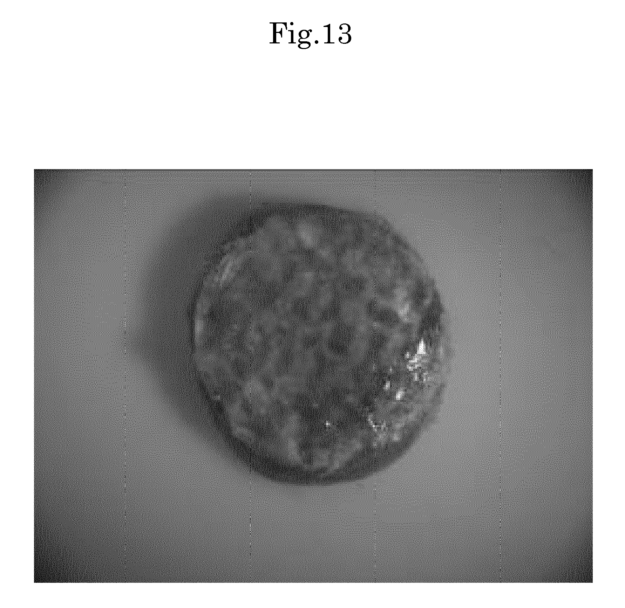

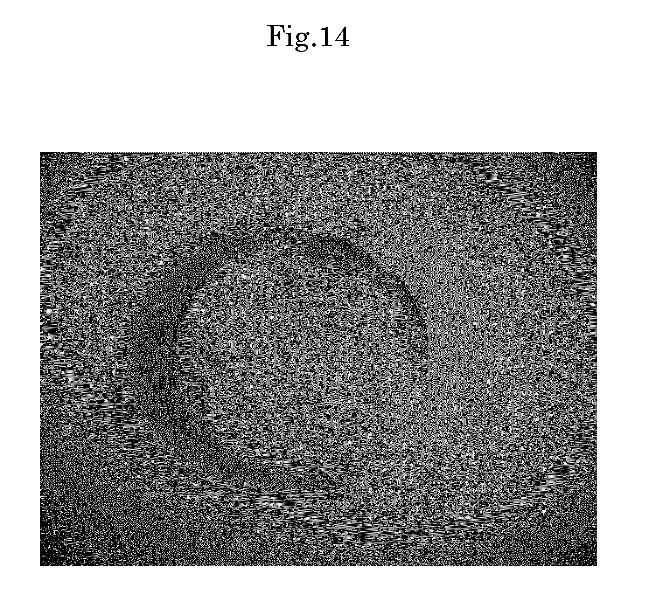

As shown in SNo.1-01, and 1-04 to 1-06, antithrombogenicity was not recognized for the disc-shaped SUS316L (SUS*), acryl*, PTFE*, and HAP* (see Figs. 1 to 4). On the other hand, the polymer layer coating of the present invention imparted significant antithrombogenicity to the base material surfaces in SNo.1-10, and 1-13 to 1-15 (see Figs. 11 to 14).

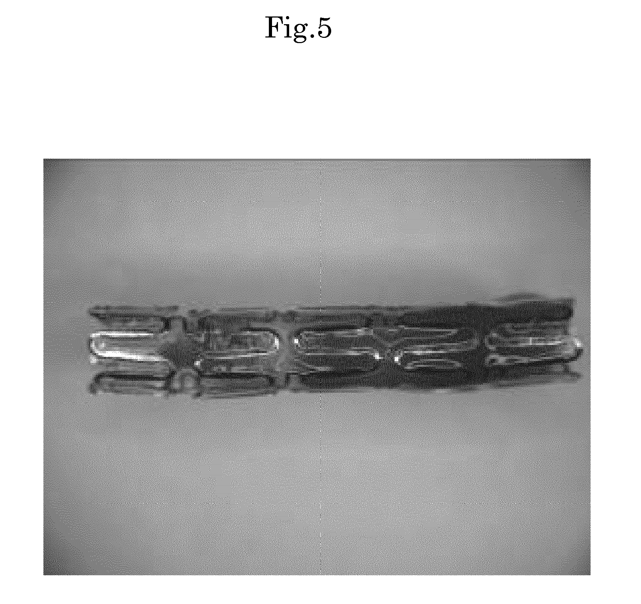

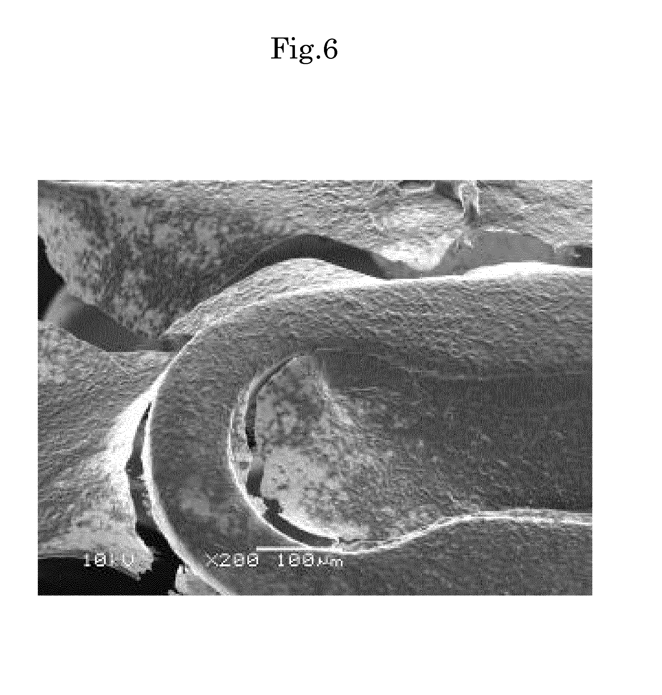

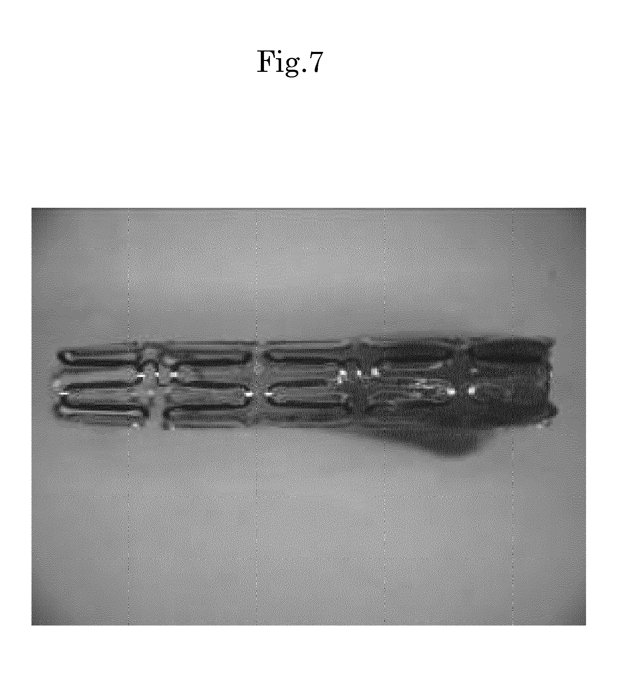

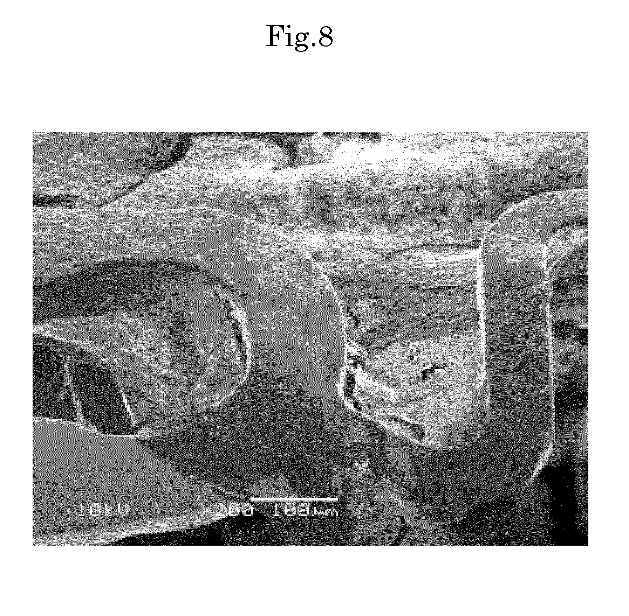

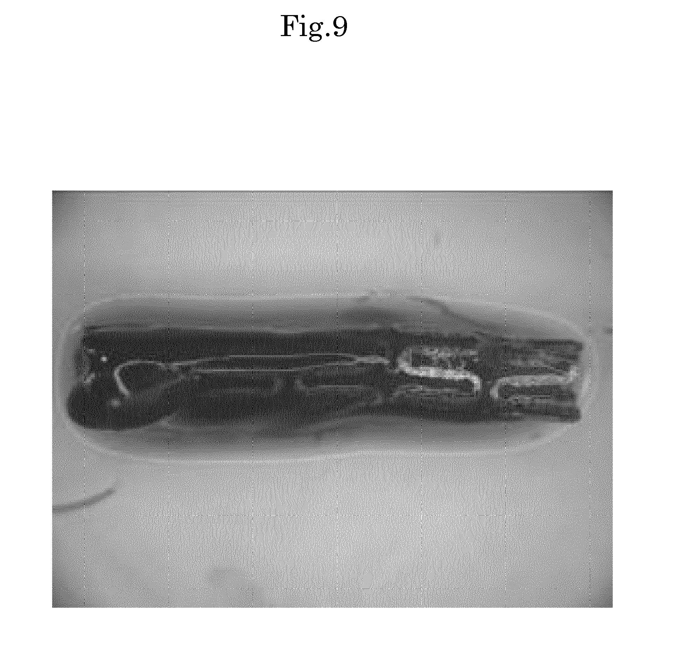

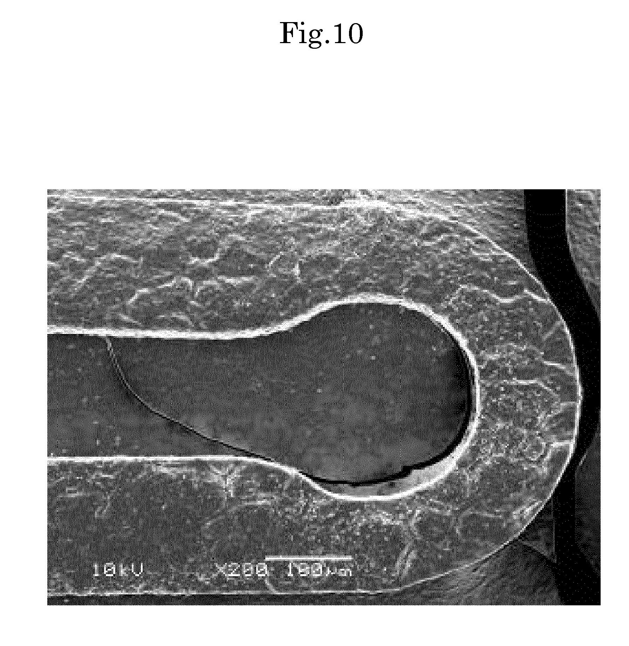

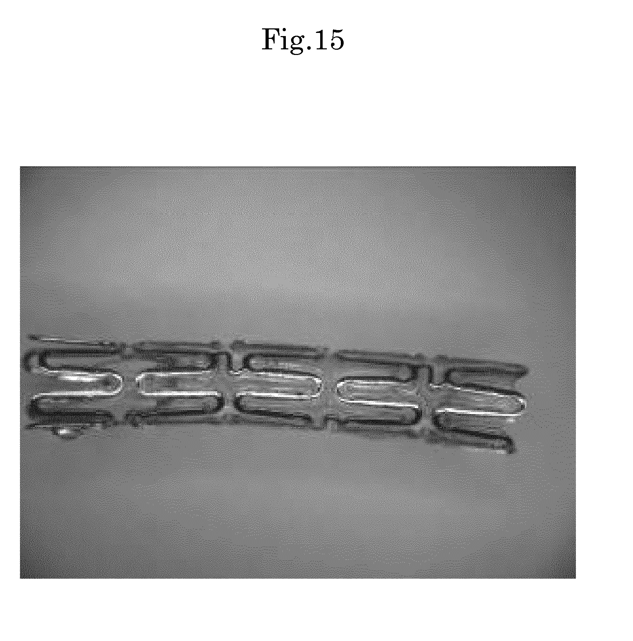

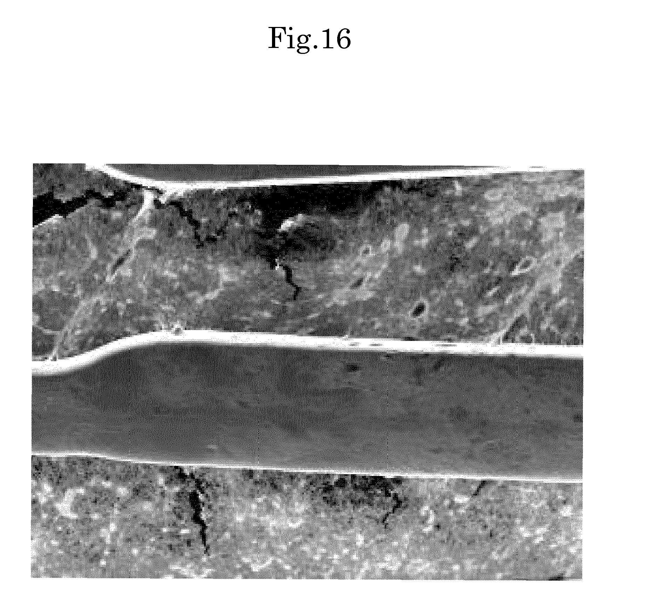

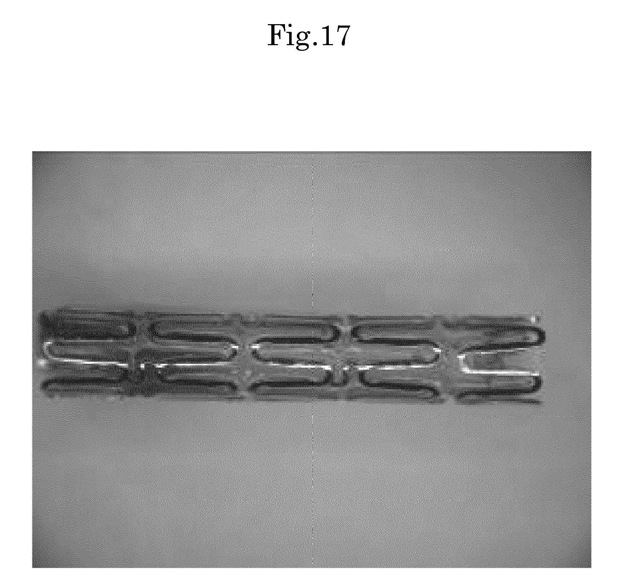

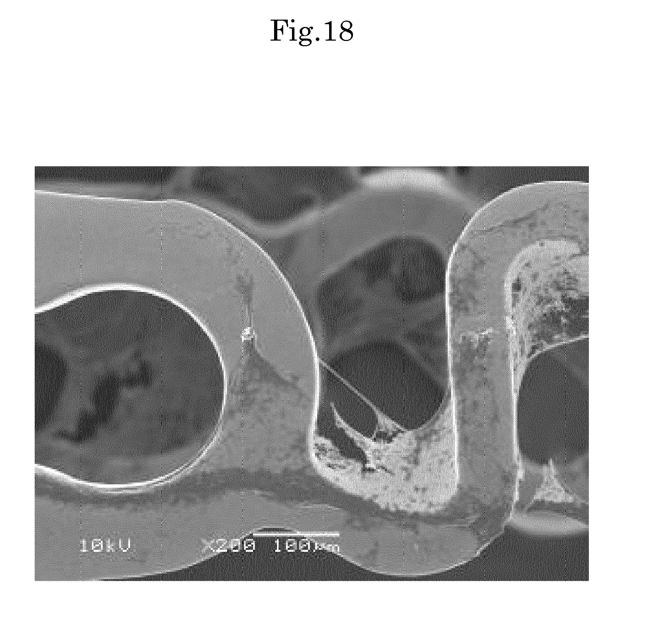

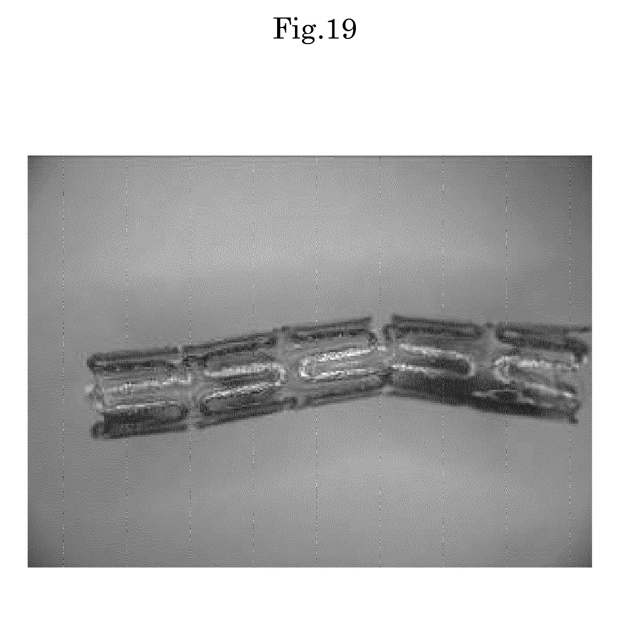

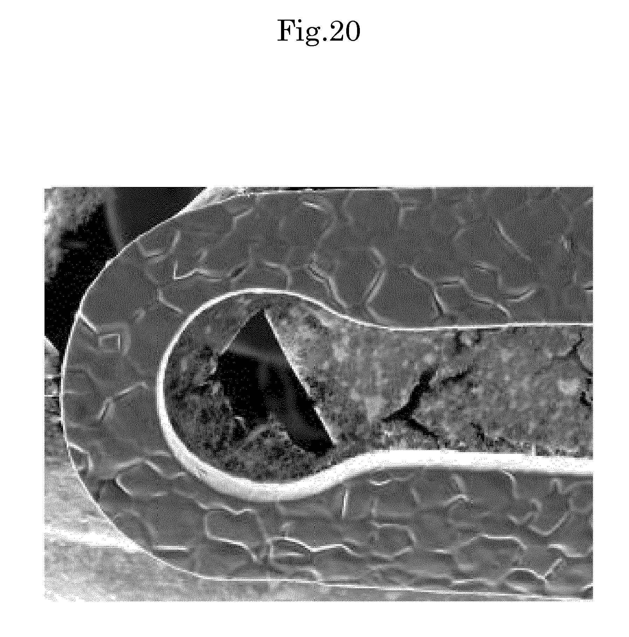

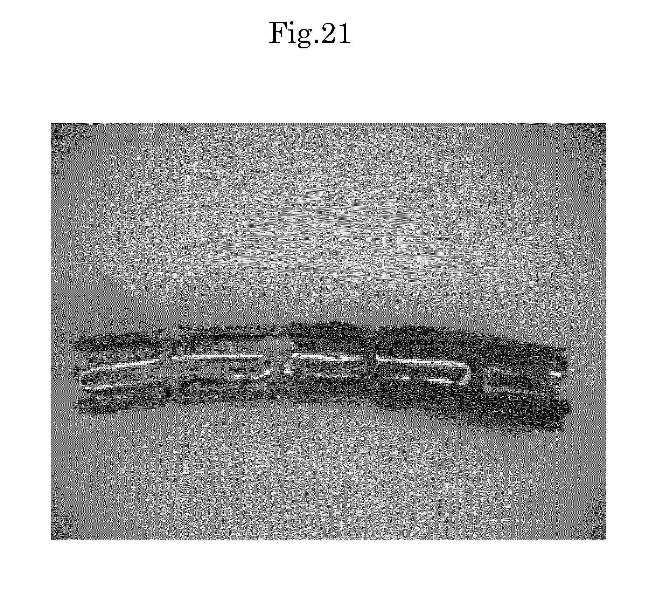

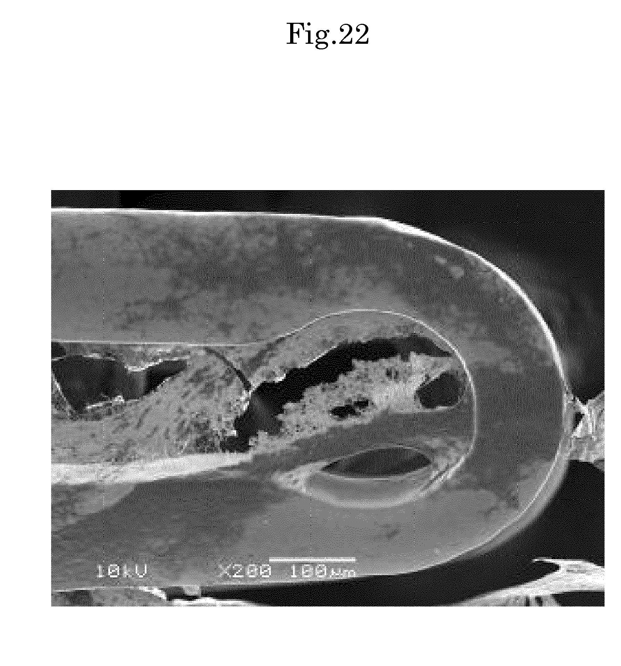

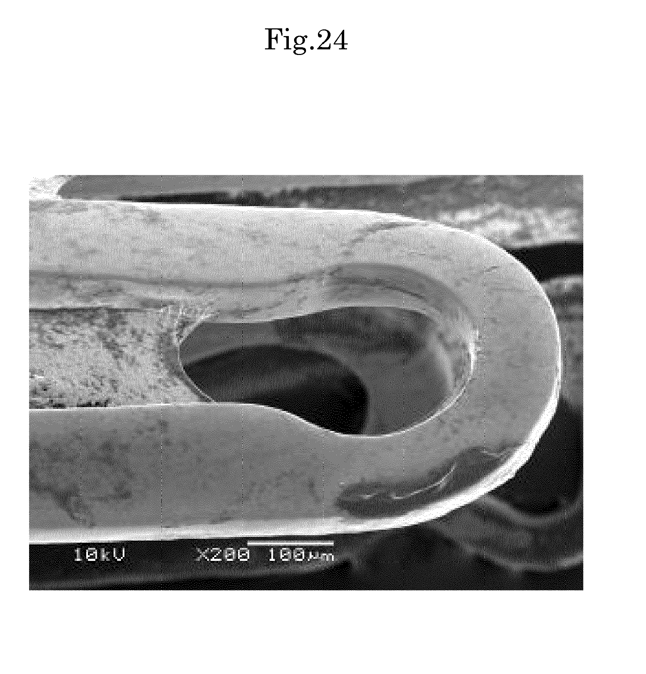

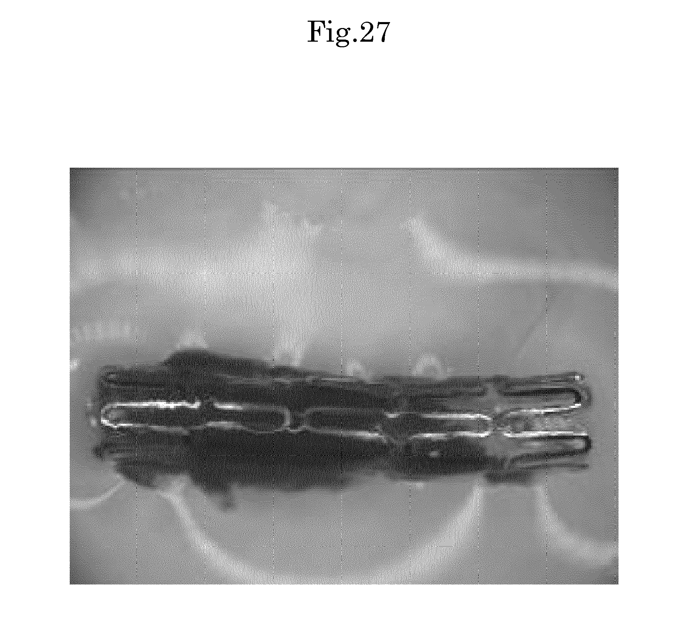

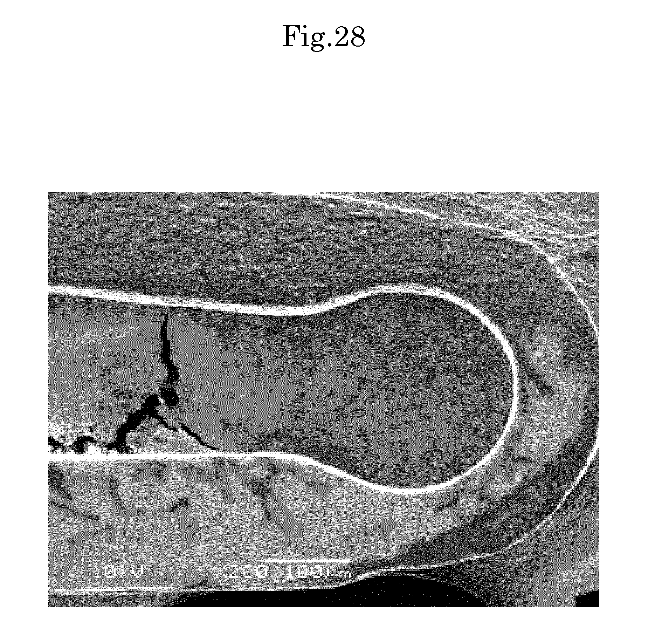

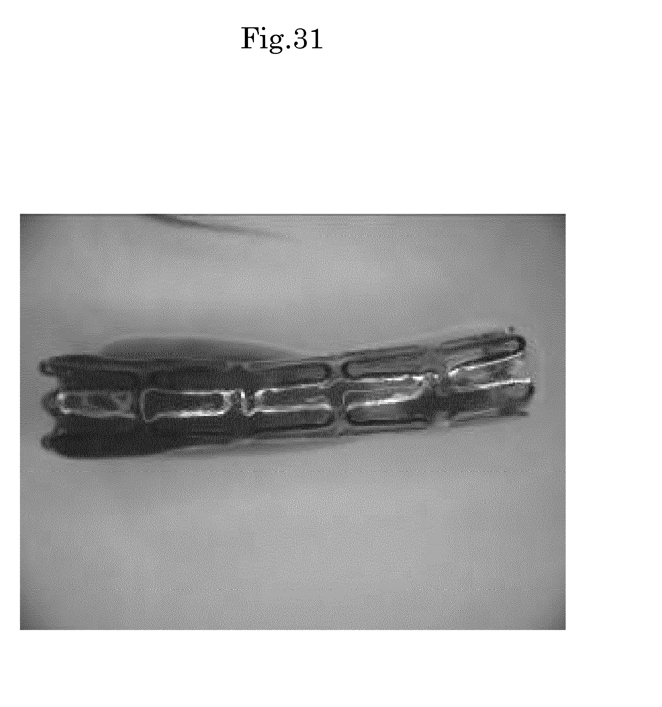

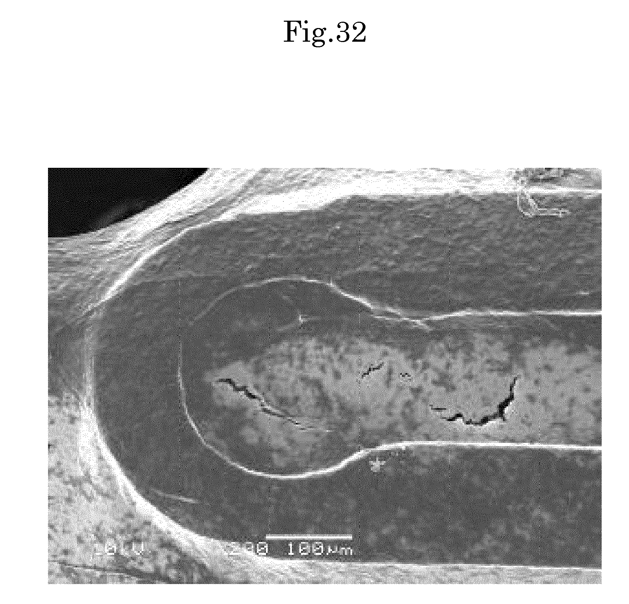

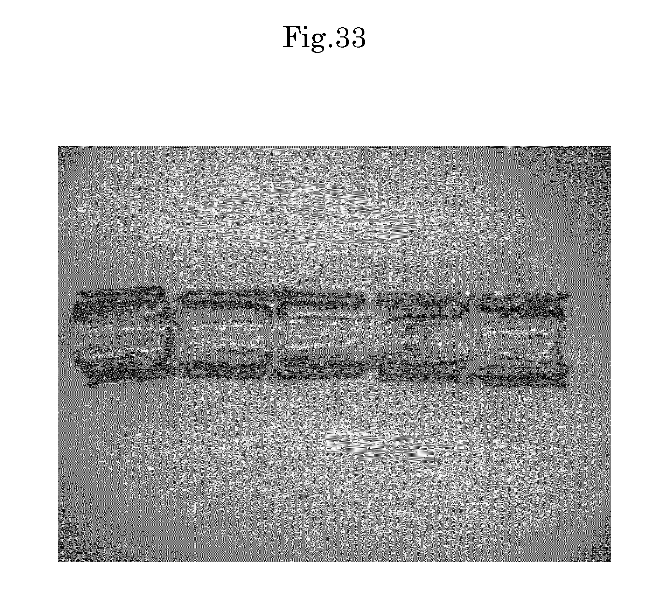

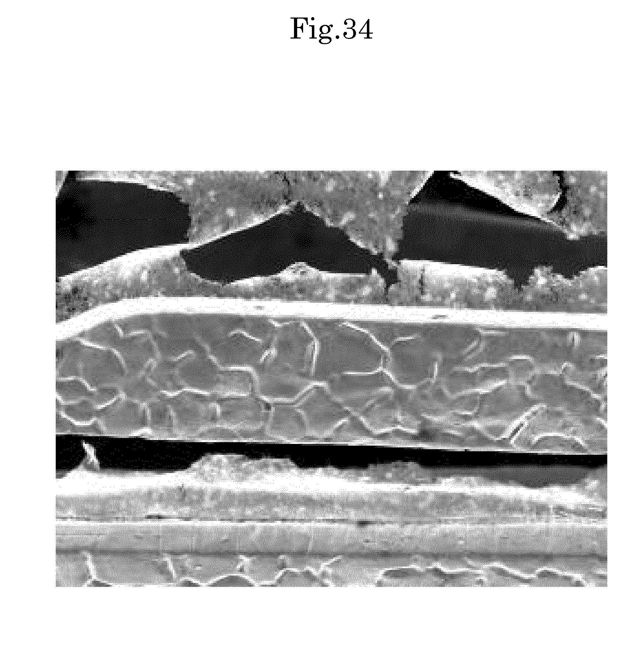

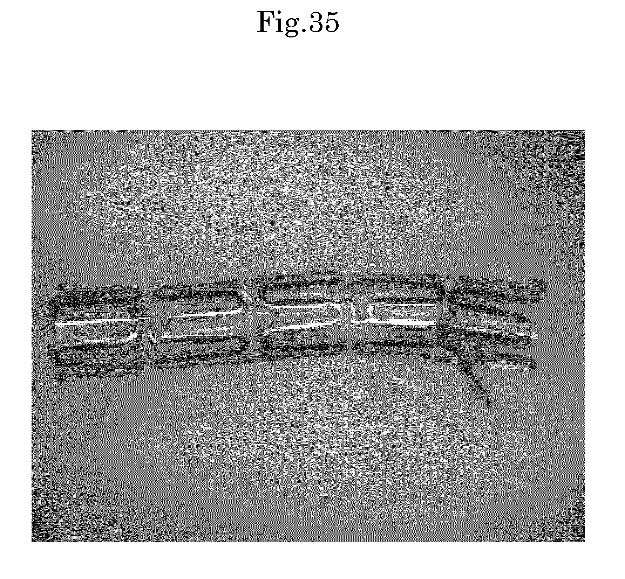

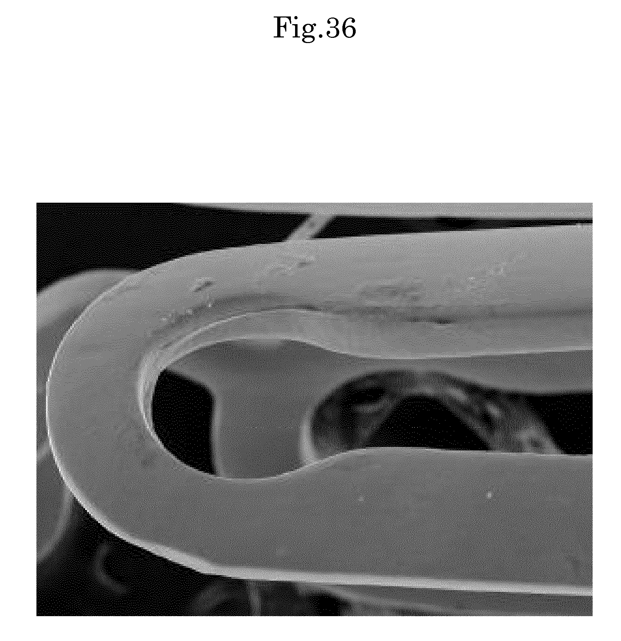

The same evaluation was performed for the SUS*, CoCr*, and HNS* stents. As shown in SNo.1-07 to 1-09, antithrombogenicity was not recognized for the stent alone, and instead formation of a fibrin network was observed on the stent strut surface and between the struts (see Figs. 5 to 10). On the other hand, the polymer layer coating of the present invention imparted antithrombogenicity in SNo.1-16 to 1-18, and blood clotting was not observed on the stent strut surface or between the struts (see Figs. 15 to 20). Some blood clotting was observed, and antithrombogenicity was not imparted in SNo.1-27 in which the CoCr* stent was coated with commercially available collagen (see Figs. 31 and 32). Evaluation of the polymer layer coatings containing the drug Am80 revealed no blood clotting, and antithrombogenicity was imparted, as shown in SNo. 1-19, 1-20, 1-28, and 1-29 (see Figs. 21 to 24, and Figs. 33 to 36). There was a slight tendency for blood clotting at a high drug Am80 concentration, as shown in SNo.1-23 (see Figs. 27 and 28).

### [Vascular Endothelial Cell Adhesion and Reendothelialization Evaluations]

Normal human umbilical vein endothelial cells (HUVEC; Lonza; 5 × 10⁵ cells/well) were inoculated on the disc-shaped base materials prepared as above. An endothelial basal medium with an endothelial cell additive factor (EGM-2 Bullet Kit; Lonza) was used as the medium. The cells were cultured at 37 °C, 5% CO₂ conditions for 1 day, and counted by using a WST-1 (Cell Counting Kit, Dojindo Molecular Technologies, Inc.) reagent. The experiment was repeated 3 to 7 times under the same conditions, and the result was given as the number of adhered cells on the base materials. The acryl* and HAP* samples were evaluated qualitatively using Giemsa staining.

As for the stents, each prepared sample was loaded in a catheter, sterilized with ethylene oxide gas, and placed in the left anterior descending coronary artery (LAD), left circumflex artery (LCX), or right coronary artery (RCA) of a 3-month old pig (a body weight of about 60 kg) under anesthesia. After 2 weeks, the blood vessel at the site of the stent was taken out, and observed for reendothelialization and blood clotting through macroscopic observation of reendothelialization and scanning electron microscopic observation of the microstructure after fixing with neutral buffered formalin.

The effects of the polymer layer coating of the present invention on the base material surfaces were evaluated for SNo.1-01 to 1-06, and 1-10 to 1-15 by inoculating the HUVEC.

In SNo.1-01 to 06 that had no polymer coating, the HUVEC adhesion to the base material was poor, even though slight adhesion was observed in HAP.

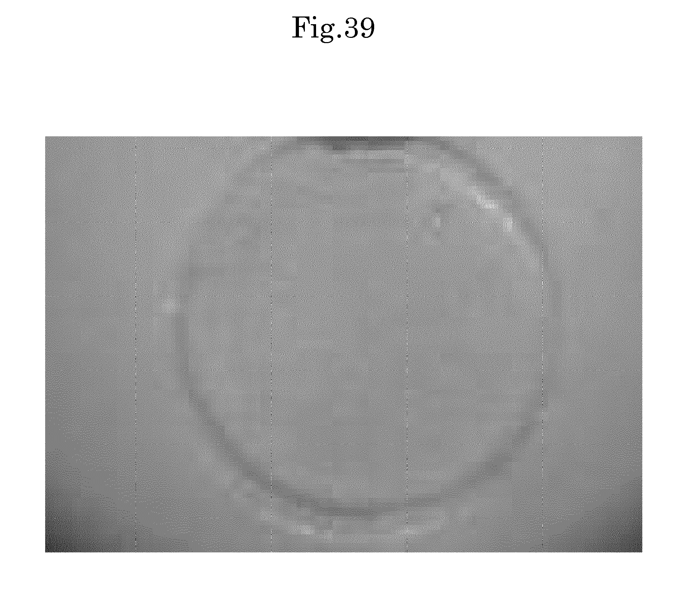

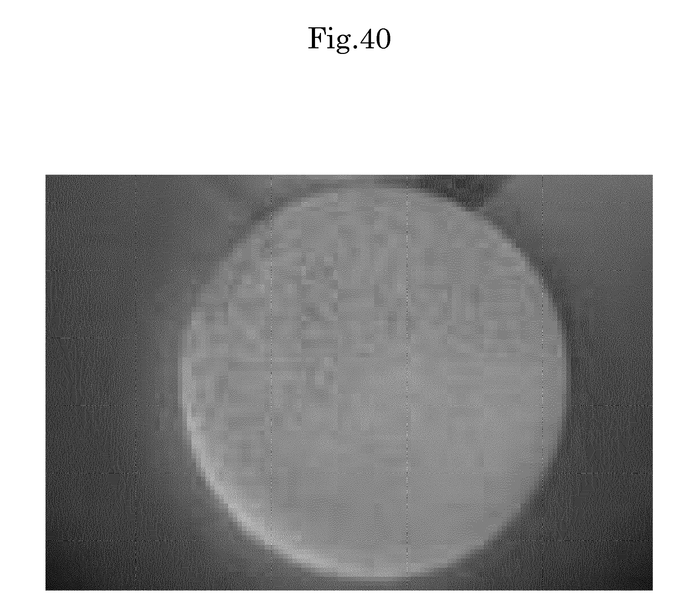

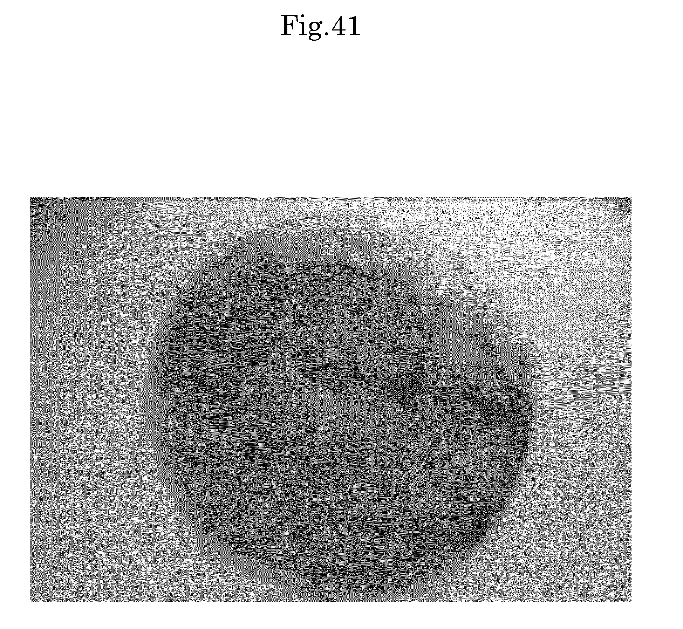

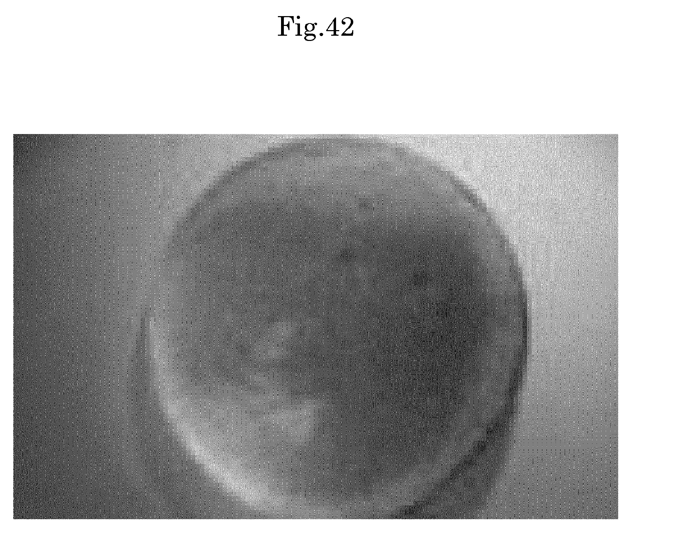

On the other hand, significant levels of HUVEC adhesion was obtained in the base materials of SNo.1-10 to 15 that had the polymer layer coatings. As can be seen in Table 2 representing the results of the quantitative evaluation of the adhered HUVEC, the polymer layer coating increased the number of adhered cells by a factor of at least 1.5 to 5 (see Figs. 39 to 42, and Table 2).

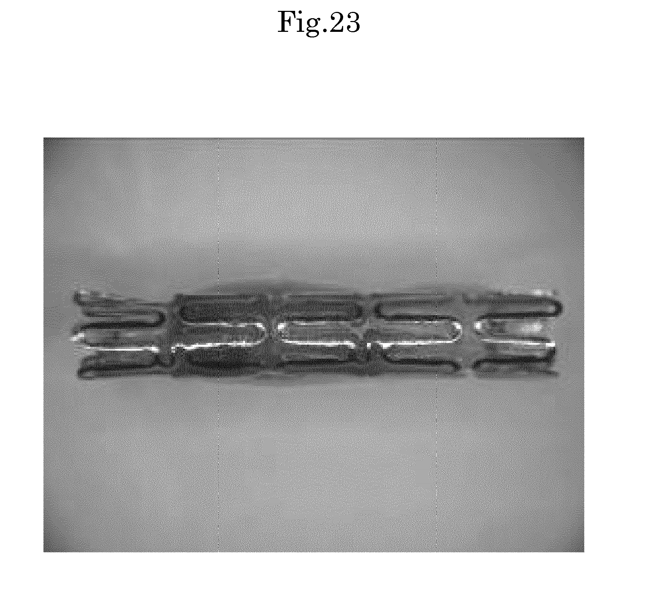

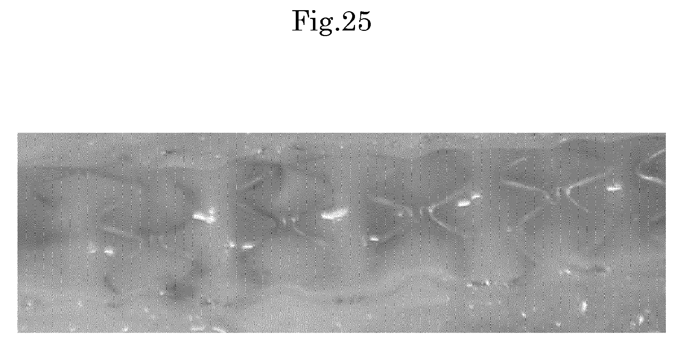

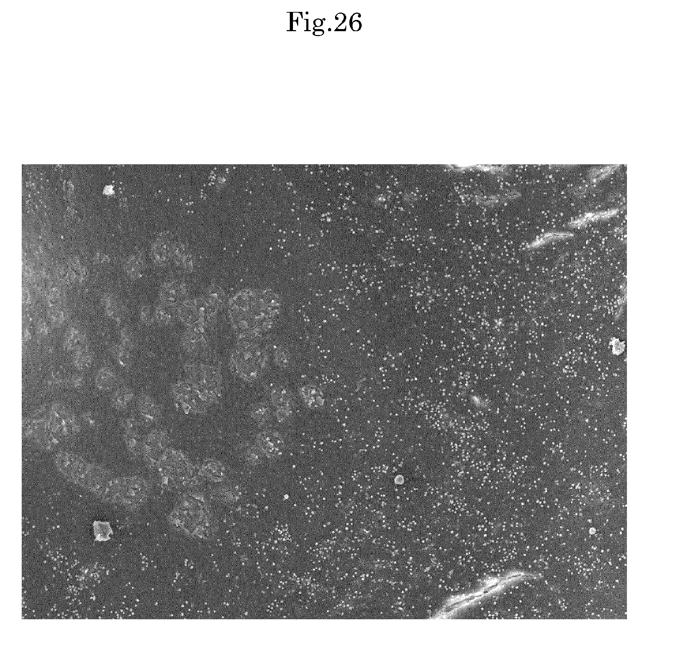

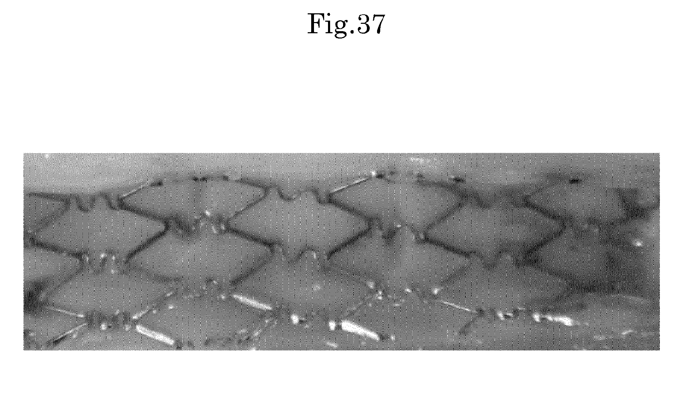

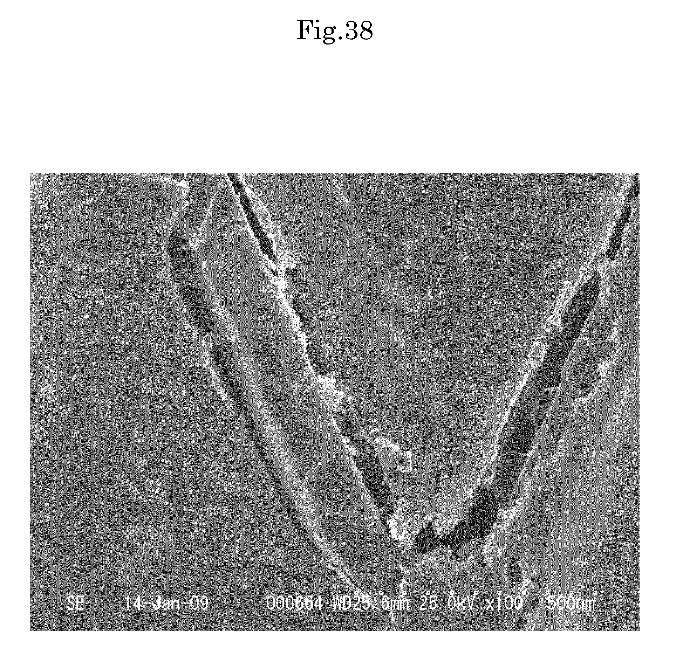

As for the stents, reendothelialization was evaluated for SNo.1-20, 1-26, and 1-30. As shown in Figs. 23 to 26, the stents with the polymer layer coating of the present invention had reendothelialization and no blood clotting, demonstrating that antithrombogenicity was maintained also in the body. On the other hand, reendothelialization was not observed, and blood clotting occurred in large numbers between the stent struts in the commercially available Cypher stent, as shown in Figs. 37 and 38. These results thus showed that the polymer layer coating of the present invention had significantly improved reendothelialization and antithrombogenicity also in the body compared to the conventional product.

### [Evaluation of Drug-releasing Property]

Stents with polymer layers containing Am80 (concentrations of 35 mM, 245 mM, and 700 mM as prepared) were heated in an 80°C oven for 10 min, and sterilized for 10 min by UV irradiation. Each sample was then dipped in 1 mL of a 0.1 M phosphate buffer (PBS; pH 7.4), and left unattended at 37°C. After a certain time period, the supernatant was sampled, and the amount of Am80 elution from the stent was quantified by high-performance liquid chromatography (HPLC). The total Am80 amount on the stent was quantified as follows. The prepared stent was dipped in 1 mL of 0.1 M PBS (pH 7.4) containing 1 mg/mL collagenase and CaCl₂, and incubated at 37°C for 24 hours to enzymatically decompose the coating layer. After filtration through a 0.2-µm filter, the filtrate was quantified by HPLC.

The sustained release of the drug Am80 from the stent was exam ined for SNo.1-19 to 25, and 28.

As presented in Table 3, a sustained release was not detected at the as-prepared drug Am80 concentration of 35 mM. However, the drug eluted over the course of 8 weeks at the concentrations of 245 mM and higher. A comparison in terms of the amount of elution after 4 weeks revealed that the amount of sustained release increases with increase in the concentration of the polymer gelatin solution even at the same as-prepared drug concentration. It was also found that the amount of sustained drug release increases with increase in the concentration of the crosslinker, TSC.

This phenomenon is suggestive of the π-π interaction or other intermolecular interactions between the polymer layer components gelatin and Am80.

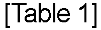

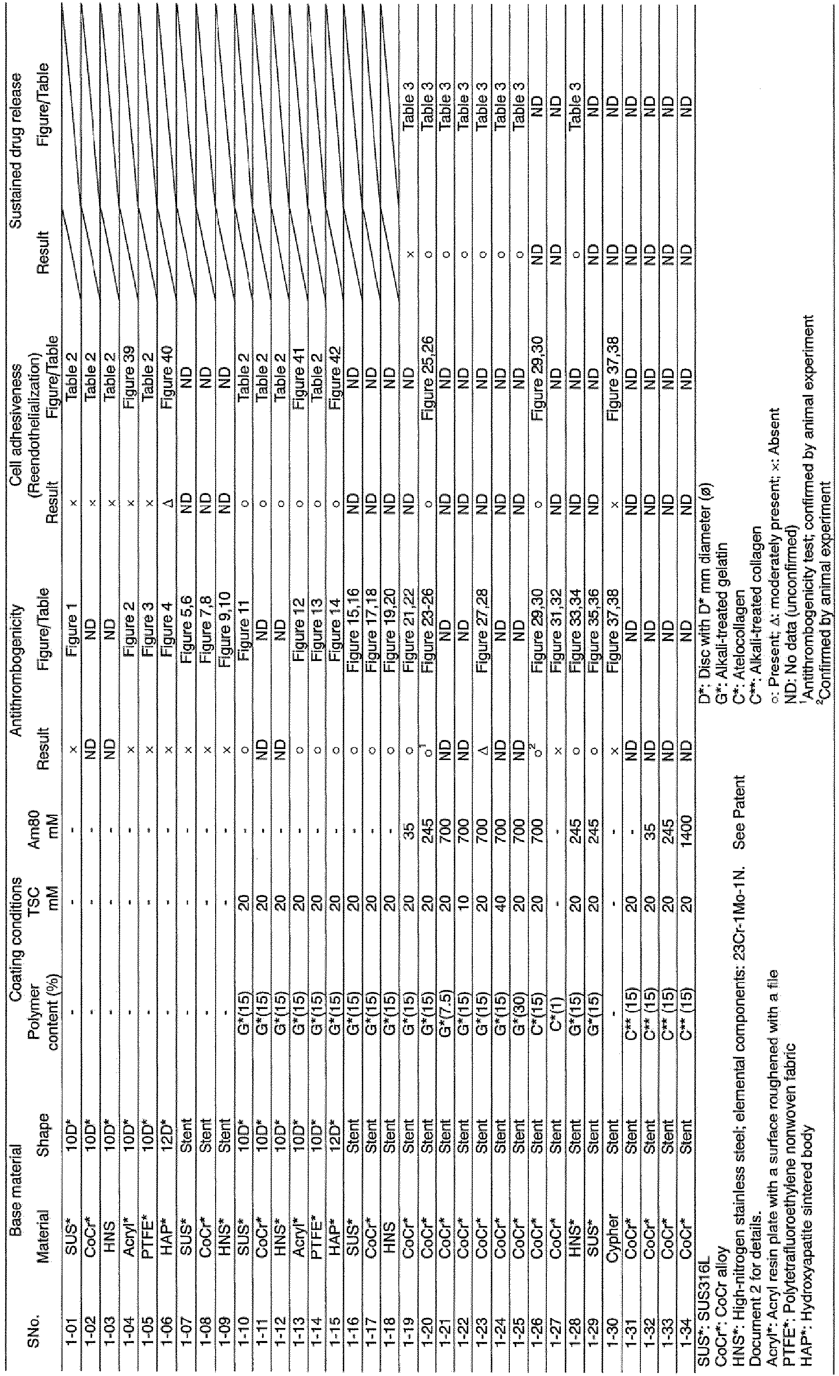

**[Table 2]**

| Base material | SNo. in Table 1 | Polymer layer | Number of adhered cells (×10³ cells) |
|---|---|---|---|
| SUS* | 1-01 | Absent | 19 |
| | 1-10 | Present | 30 |
| HNS | 1-03 | Absent | 6 |
| | 1-12 | Present | 30 |
| CoCr* | 1-02 | Absent | 10 |
| | 1-11 | Present | 24 |
| PTFE* | 1-05 | Absent | 24 |
| | 1-14 | Present | 57 |
| SUS*: SUS316L | | | |
| CoCr*: CoCr alloy | | | |
| PTFE*: Polytetrafluoroethylene nonwoven fabric | | | |

**[Table 3]**

| Elapsed time (Weeks) | | Amount of sustained release of drug Am80 (µg) | | | | | | | |
|---|---|---|---|---|---|---|---|---|---|
| | SNo. in Table 1 | 1-19 | 1-20 | 1-21 | 1-22 | 1-23 | 1-24 | 1-25 | 1-28 |
| 1 | Solid No 1 | Undetected | 0.81 | - | - | 4.00 | - | - | 1.53 |
| | Solid No 2 | Undetected | 0.65 | - | - | 4.39 | - | - | - |
| | Solid No 3 | Undetected | 0.71 | - | - | 3.34 | - | - | - |
| | Average of 1 to 3 | Undetected | 0.72 | - | - | 3.91 | - | - | 1.53 |
| 2 | Solid No 1 | Undetected | 0.70 | - | - | 7.68 | - | - | 1.67 |
| | Solid No 2 | Undetected | 0.82 | - | - | 4.99 | - | - | - |
| | Solid No 3 | Undetected | 0.75 | - | - | 4.42 | - | - | - |
| | Average of 1 to 3 | Undetected | 0.76 | - | - | 5.70 | - | - | 1.67 |
| 4 | Solid No 1 | Undetected | 0.81 | 0.70 | 5.51 | 4.83 | 8.52 | 29.90 | 1.88 |
| | Solid No 2 | Undetected | 0.69 | 0.88 | 15.29 | 6.76 | 12.19 | 37.96 | - |
| | Solid No 3 | Undetected | 0.92 | 1.67 | 3.17 | 6.81 | 12.84 | 49.67 | - |
| | Average of 1 to 3 | Undetected | 0.81 | 1.08 | 7.99 | 6.14 | 11.18 | 39.18 | 1.88 |
| 8 | Solid No 1 | Undetected | 0.94 | - | - | 6.16 | - | - | 2.05 |
| | Solid No 2 | Undetected | 0.79 | - | - | 7.42 | - | - | - |
| | Solid No 3 | Undetected | 0.81 | - | - | 10.93 | - | - | - |
| | Average of 1 to 3 | Undetected | 0.85 | - | - | 8.17 | - | - | 2.05 |

### Example 2

### [Verification of Restenosis Suppressing Effect]

A stent prepared as above was placed in a left anterior descending artery (LAD), a left circumflex artery (LCX), or a right coronary artery (RCA). A maximum of two stents were placed in the LAD, LCX, and RCA. After 4 weeks, the extent of restenosis was examined by quantitative coronary angiography (QCA), and evaluated according to the AHA (American Heart Association) classification of coronary artery.

According to the AHA classification, the percentage coarctation is classified as follows: 0% for no coarctation; 25% for 1 to 25% coarctation; 50% for 25 to 50% coarctation, 75% for 51 to 75% coarctation, 90% for 75 to 99% coarctation (no slowing of a dye flow at the site of constriction), 99% for 75 to 99% coarctation (slowing of a dye flow at the site of constriction) and 100% for complete coarctation.

Five to nine stents were placed for each condition, and the proportion of samples with a restenosis rate of 45% or less was calculated. Samples with a 60% or higher proportion were deemed as having the restenosis suppressing effect. A CoCr bare metal stent (CoCr-BMS), a SUS316L bare metal stent (SUS-BMS), and a Cypher stent (Johnson & Johnson) were used as Comparative Examples.

The effectiveness of the polymer layer coating (4-01) and the Am80-containing polymer layer coatings (4-04 to 07) was confirmed as compared with CoCr-BMS (4-08), SUS-BMS (4-09), and Cypher (4-10).

**[Table 4]**

| SNo. | SNo. in Table 1 | Coating conditions | | | | Results of recurrent stenosis evaluation | | | |
|---|---|---|---|---|---|---|---|---|---|
| | | Material | Polymer | TSC | Am80 | Percentage recurrent stenosis by sample | | | Number of effective solids* : percentage effectiveness*: effectiveness* |
| | | | Content (%) | mM | mM | | % | | |
| 4-01 | 1-31 | CoCr* | C** (15) | 20 | - | 25 | 25 | 75 | 4:80:O |
| | | | | | | 25 | 25 | | |
| 4-02 | 1-32 | CoCr* | C** (15) | 20 | 35 | 37 | 53 | 88 | 2:40:× |
| | | | | | | 37 | 57 | | |
| 4-03 | 1-33 | CoCr* | C** (15) | 20 | 245 | 0 | 42 | 54 | 4:44:× |
| | | | | | | 12 | 51 | 56 | |
| | | | | | | 36 | 53 | 58 | |
| 4-04 | 1-26 | CoCr* | C*(15) | 20 | 700 | 0 | 20 | 42 | 7:78:O |
| | | | | | | 7 | 41 | 50 | |
| | | | | | | 15 | 42 | 60 | |
| 4-05 | 1-34 | CoCr* | C** (15) | 20 | 1400 | 7 | 17 | 50 | 4:80:O |
| | | | | | | 12 | 40 | | |
| 4-06 | 1-20 | CoCr* | G*(15) | 20 | 245 | 9 | 32 | 46 | 4:80:O |
| | | | | | | 19 | 32 | | |
| 4-07 | 1-23 | CoCr* | G*(15) | 20 | 700 | 8 | 22 | 56 | 3:60:O |
| | | | | | | 15 | 55 | | |
| 4-08 | 1-08 | CoCr* | - | - | - | 23 | 47 | 59 | 3:33:× |
| | | | | | | 34 | 52 | 62 | |
| | | | | | | 43 | 53 | 100 | |
| 4-09 | 1-07 | SUS* | - | - | - | 75 | 90 | 25 | 1:20:× |
| | | | | | | 75 | 50 | | |
| 4-10 | 1-30 | Cypher | - | - | - | 9 | 46 | 91 | 3:33:× |
| | | | | | | 13 | 83 | 93 | |
| | | | | | | 25 | 83 | 96 | |
| SUS*: SUS316L | | | | | | | | | |
| CoCr*: CoCr alloy | | | | | | | | | |
| G*: Alkali-treated gelatin | | | | | | | | | |
| C*: Atelocollagen | | | | | | | | | |
| C**: Alkali-treated collagen | | | | | | | | | |
| Number of effective samples *: The number of solids with percentage recurrent stenosis of 45% or greater | | | | | | | | | |
| Percentage effectiveness*: Percentage of the number of effective samples in the total number of samples in each experiment (SNo.) | | | | | | | | | |
| Effectiveness*: O: determined effective to prevent recurrent stenosis; x: determined otherwise | | | | | | | | | |

### Example 3

### [Effect of Pretreatment for Improving Interface Adhesion between Base Material and Coating Layer]

For the purpose of strongly bonding the polymer coating layer and the metal without introducing an anchor molecule such as a silane coupling agent to the metal surface, the adhesion at the interface between the base material and the coating was examined by measuring the force at which detachment occurs at the metal-polymer layer interface. The base material was surface treated by dipping the surface (a disc with ø = 10 mm, and a thickness of 1 mm) in an organic solvent (acetone: AS), diluted aqua regia (50% aqua regia: AR), or a 10% sodium hydroxide aqueous solution (Na) for 1 hour, before being coated with the polymer layer. The surface of the base material subjected to the surface treatment was then coated with the coatings presented in Table 3, dipped in water for 3 days, and dried to obtain each sample presented in Table 5. The sample was placed between fixtures from above and below via a fast-acting adhesive, and one of the fixtures was pulled upward to measure the stress needed to detach the fixture.

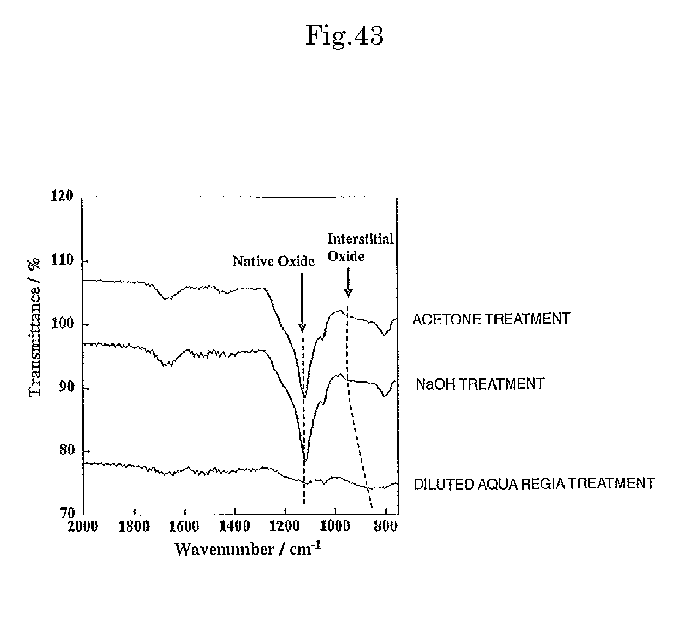

Table 5 presents the detachment strengths between the base material and the coating polymer layer on different treated surfaces. It was found that the polymer layer was attached most effectively under the acid treatment condition using the diluted aqua regia, rather than using the organic solvent or alkali treatment. In order to clarify the differences in detachment strength arising from the surface treatment, an attenuated total reflection infrared absorption spectral (ATR-IR) measurement was performed for the substrates subjected to the various pretreatments. As represented in Fig. 43, while absorption for the native oxide film was confirmed at 1,160 cm⁻¹ in the substrates treated with acetone and NaOH, a peak for the native oxide film attenuated in the substrate treated with the diluted aqua regia. Specifically, the result suggests that the crystal grain boundary that appeared as a result of the reduced oxide coating on the substrate surface after the diluted aqua regia treatment may be a factor promoting the increased detachment strength.

**[Table 5]**

| SNo. | SNo. in Table 1 | Coating conditions | | | | | Detachment strength (kPa) | | | |
|---|---|---|---|---|---|---|---|---|---|---|
| | | Material | Polymer content (%) | TSC mM | Am80 mM | Treatment condition | 1 | 2 | 3 | Average |
| 5-01 | 1-10 | SUS* | G*(15) | 20 | - | AS* | 367 | 355 | 301 | 341 |
| 5-02 | 1-10 | SUS* | G*(15) | 20 | - | Na* | 341 | 289 | 359 | 330 |
| 5-03 | 1-10 | SUS* | G*(15) | 20 | - | AR* | 813 | 808 | 767 | 796 |
| SUS*:SUS316L | | | | | AS*: Organic solvent (acetone) | | | | | |
| G*: Alkali-treated gelatin | | | | | Na*: Alkali (10% NaOH aqueous solution) | | | | | |
| | | | | | AR*: Acid (diluted aqua regia (50% aqua regia)) | | | | | |

### Example 4

Assessment was made as to the effect of the number of polymer layer coating cycles on the base material surface. Specifically, a solution prepared by adding tamibarotene (Am80) in 35 mM to a 10% alkali-treated gelatin (AlGltn)/hexafluoroisopropanol (HFIP) solution that contained 13 mM TSC as the crosslinker was used to form a coating multiple times on a stent surface.

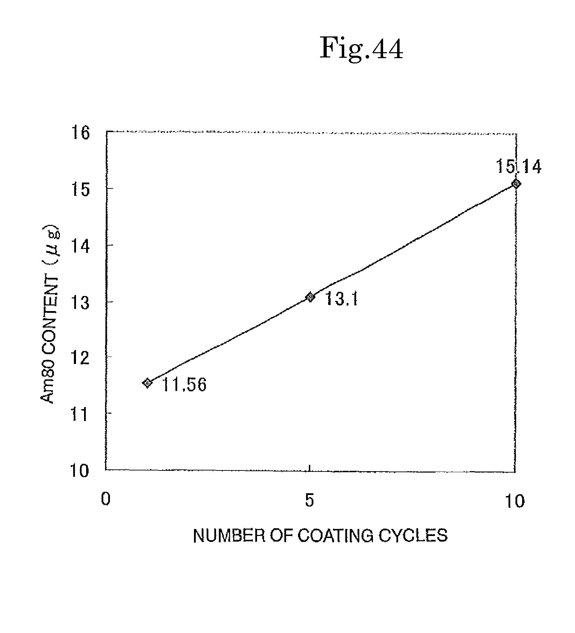

The results are represented in Fig. 44. As shown in Fig. 44, the Am80 content increased almost linearly with increase in the number of coating cycles from 1 to 5 and to 10. It was confirmed from this that the Am80 content can be controlled by controlling the number of coating cycles.

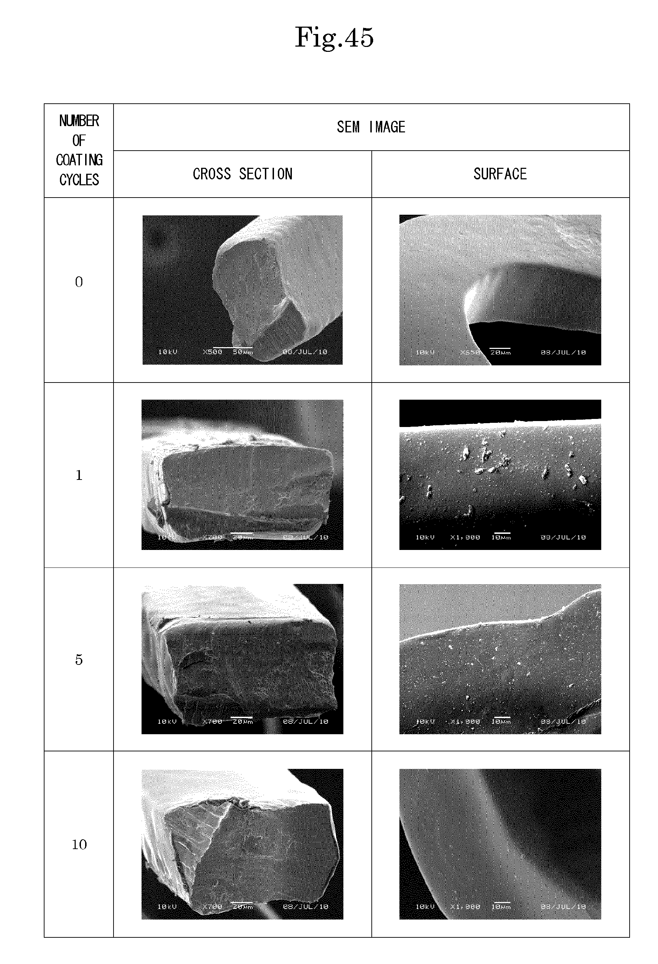

Fig. 45 represents SEM images of a stent surface and a stent cross section after the coating. As shown in Fig. 45, it was confirmed that the thickness of the polymer layer on the base material surface increases proportionally to the number of coating cycles. After 10 cycles of coating, the polymer layer coating had a thickness of 2 to 3 µm.

### Example 5

The polymer layer coated on the base material surface was examined under different coating solution conditions and coating conditions to evaluate the effects of these conditions on the sustained release (elution amount) of the drug contained in the polymer layer.

A 10% alkali-treated gelatin and a crosslinker (TSC) were coated on a stent surface to form a polymer layer under the conditions presented in Table 6. Tamibarotene (Am80) was used as the drug, and the concentration in the polymer layer was adjusted to 35 mM. The prepared stent was dipped in 1 mL of 0.1 M phosphate buffer (pH 7.4) at 37°C, and the elution amount of Am80 after 7 days was checked.

The results are presented in Table 6.

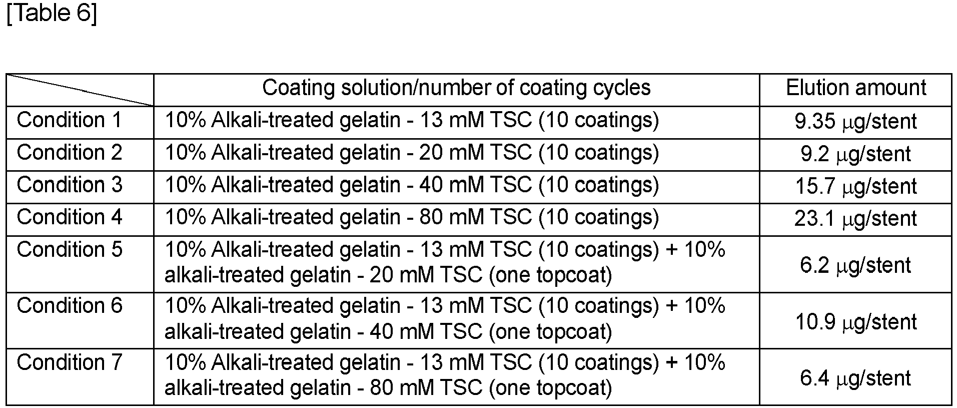

The results for conditions 1 to 4 confirmed that the sustained drug release had the tendency to increase with increasing crosslinker contents. It can also be seen from the results for conditions 5 to 7 that the presence of a top coat has only a little effect on the elution amount.

### Example 6

Assessment was made as to a method of controlling the elution time of the drug from the polymer layer coated on a base material surface.

Gelatin was used as the cell-adhesive peptide-containing polymer. The gelatin was hydrophobically modified by partially modifying the amino group in the gelatin with a retinoyl (RA) group or an eicosapentaenoic (EPA) group to examine whether such modification can control the sustained release of Am80.

### <1> Synthesis of Hydrophobically modified Gelatin

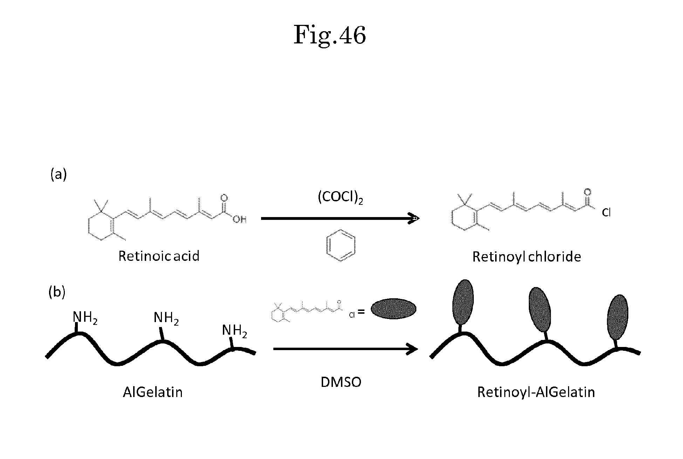

1) The amino group in the gelatin was hydrophobicacally modified with an eicosapentaenoic group in 50 %. (Hereinafter, the hydrophobized gelatin will be referred to as E50G.)
2) The amino group in the gelatin was chemically modified with a retinoyl group in 0, 25, 50, and 75%. The synthesis scheme of the retinoylated gelatin is represented in Fig. 46. (In the following, the hydrophobically modified gelatins will be referred to as R0G, R25G, R50G, and R75G.)

### <2> Preparation of Drug Containing-Polymer Matrix

Tamibarotene (Am80) was used as the drug, and a 10% RG (E50G, R0G, R25G, R50G, and R75G)/DMSO solution containing Am80 (35 mM) was prepared. Then, a TSC/DMSO solution was added to make the final concentration 13 mM. After molding the mixture into a plate shape, the plate was dipped in water (4°C) for 72 hours. After replacing the DMSO with water and removing the by-product, the product was freeze dried to obtain a dry Am80-containing matrix.

### <3> Elution of Am80 from Am80-Containing Matrix

About 1 mg of the dry Am80-containing matrix was weighed into a 50-mL conical tube, and allowed to stand at 37°C after adding 50 mL of 0.1 M PBS (pH 7.4). After 7 days, the eluate (1 mL) was taken out, and used as a HPLC sample to examine the Am80 elution amount per gram of the matrix after 7 days, and the percentage Am80 release from the matrix after 7 days. Details of the HPLC measurement conditions are as follows.

HPLC measurement conditions
Column: Nacalai Tesque COSMOSIL PACKED COLUMN (Size: 4.6 I.D. × 150 mm; Type: 5C18-AR-II WATERS)
Mobile phase: 5% HOAc/CH₃CN = 35/65 (v/v)
Column temperature: 40.0°C
Flow rate: 1.00 mL/min
Injection amount: 10.0 µL
Detection wavelength: 286 nm

### <4> Results

The results are presented in Table 7. The percentage Am80 release in Table 7 was calculated for each condition (R0G, R25G, R50G, R75G, E50G) relative to the 100% Am80 amount contained in the cell-adhesive peptide-containing polymer (gelatin) of the stent before the sustained-release test.

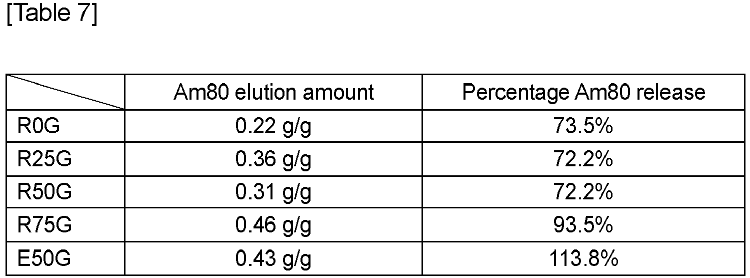

As presented in Table 7, gelatin hydrophobization enabled the elution amount and the release rate of the drug (Am80) to be controlled. It was therefore confirmed that a biocompatible device using a hydrophobically modified cell-adhesive peptide-containing polymer can be used to control the elution time (sustained release) of the drug according to such factors as the type of the drug, and the conditions of the user using the biocompatible device.

### Industrial Applicability

The present invention is also applicable as a diagnosis material upon coating the base material with the polymer layer in patterns.

## Claims

1. A biocompatible device surface-coated on the base material thereof with a biocompatible polymer layer having antithrombogenicity and endothelialization activity, and embedded in or attached to a living body for use, wherein the polymer layer comprises a polymer matrix formed by the crosslinking of a cell-adhesive peptide-containing polymer.

2. The biocompatible device according to claim 1, wherein the polymer matrix is formed by the crosslinking of the polymer via a citric acid derivative with active ester groups.

3. The biocompatible device according to claim 2, wherein the citric acid derivative with active ester groups is trisuccinimidyl citrate or trisulfosuccinimidyl citrate.

4. The biocompatible device according to any one of claims 1 to 3, wherein the cell-adhesive peptide-containing polymer is one or a combination of two or more selected from gelatin, alkali-treated gelatin, acid-treated gelatin, collagen, atelocollagen, alkali-treated collagen, fibrinogen, keratin, fibroin, laminin, fibronectin, vitronectin, and a derivative thereof.

5. The biocompatible device according to any one of claims 1 to 4, wherein the cell-adhesive peptide represents one of or a combination of two or more of the peptide sequences selected from arginine-glycine-aspartic acid (RGD), tyrosine-isoleucine-glycine-serine-arginine (YIGSR), and isoleucine-lycine-valine-alanine-valine (IKVAV).

6. The biocompatible device according to any one of claims 1 to 5, wherein the base material is one or a composite material of two or more selected from a polymer material, a metallic material, a ceramic material, a nonwoven fabric, and a biological tissue.

7. The biocompatible device according to claim 6, wherein the base material is a polymer material, and wherein the polymer material is one or a combination of two or more selected from polyethylene, polypropylene, polytetrafluoroethylene, polystyrene, polyurethane, silicone, polylactic acid, polyglycolic acid, poly ε-caprolactone, a polylactic acid-glycolic acid copolymer, a poly ε-caprolactone-glycolic acid copolymer, and a polylactic acid-poly ε-caprolactone copolymer.

8. The biocompatible device according to claim 6, wherein the base material is a metallic material, and wherein the metallic material is one or a combination of two or more selected from SUS316L stainless steel, a cobalt-chromium alloy, nickel-free high-nitrogen stainless steel, a magnesium alloy, and a shape-memory alloy.

9. The biocompatible device according to claim 6, wherein the base material is a ceramic material, and wherein the ceramic material is one or a combination of two or more selected from a hydroxyapatite sintered body, low crystalline hydroxyapatite, β-tricalcium phosphate, and α-tricalcium phosphate.

10. The biocompatible device according to any one of claims 1 to 9, wherein the polymer layer is formed on a base material surface surface-treated with an acid, an alkali, or an organic solvent.

11. The biocompatible device according to any one of claims 1 to 10, wherein a drug is included in the polymer matrix.

12. The biocompatible device according to claim 11, wherein the drug is one or a combination of two or more selected from a cellular differentiation inducer, an anticancer agent, an immunosuppresant, a cell growth factor, a cytokine, a thrombin inhibitor, an antithrombogenic drug, a thrombolytic agent, a fibrinolytic drug, a vasospasm inhibitor, a calcium channel blocker, a vasodilating drug, a high blood pressure drug, an antimicrobial drug, an antibiotic, a surface glycoprotein receptor inhibitor, an anti-platelet drug, an antimitotic drug, a microtubule inhibitor, an antisecretory drug, an actin inhibitor, a remodeling inhibitor, an antisense·nucleotide, an antimetabolite, an antiproliferative substance, an anti-cancer chemotherapy drug, an anti-inflammatory steroid or a nonsteroidal anti-inflammatory drug, an immunosuppresant, a growth hormone·antagonist, a growth factor, a dopamine agonist, a radiotherapeutic agent, a peptide, a protein, an enzyme, an extracellular matrix component, an inhibitor, a free radical·scavenger, a chelating agent, an antioxidizing agent, an anti-polymerase, an anti-viral drug, a photodynamic therapeutic drug, and a gene therapy drug.

13. The biocompatible device according to claim 12, wherein the cellular differentiation inducer is tamibarotene.

14. A medical apparatus embedded in or attached to a living body for use in a medical procedure, the medical apparatus comprising the biocompatible device of any one of claims 1 to 13 configured to have a structure suited for the medical procedure.

15. A stent inserted into a blood vessel of a living body to dilate the blood vessel from inside, the stent comprising the biocompatible device of any one of claims 1 to 14 configured to have the structure of the stent.

16. The biocompatible device according to claim 10, wherein the acid is aqua regia.

17. The biocompatible device according to claim 4, wherein the cell-adhesive peptide-containing polymer is hydrophobically modified.

18. A biocompatible device producing method, comprising coating a surface of a base material with a coating solution that contains a cell-adhesive peptide-containing polymer and a crosslinker, so as to form a polymer layer having antithrombogenicity and endothelialization activity.

19. The biocompatible device producing method according to claim 18, wherein the coating is performed multiple times.

20. The biocompatible device producing method according to claim 18, wherein the coating solution contains a drug.

21. The biocompatible device producing method according to claim 18, further comprising the hydrophobically modified cell-adhesive peptide-containing polymer.

22. The biocompatible device producing method according to claim 18, wherein the coating solution contains a drug, and wherein the method further comprises adjusting the concentration of the crosslinker in the coating solution in a range of from 5 mM to 200 mM according to the desired drug sustained-release from the biocompatible device.
